# EUROPÄISCHE PATENTANMELDUNG

(11) **EP 1 167 529 A2**
(43) Veröffentlichungstag der Anmeldung: **02.01.2002**
(21) Anmeldenummer: 01109460.4
(22) Anmeldetag: 24.04.2001
(51) Int. Cl.: C12N 15/79, C12N 15/08, C12N 1/21

(54) **Vektorsystem zum Einbringen von DNA in eukaryonte Zellen**

(30) Priorität: 26.04.2000 DE 10020500
(71) Anmelder: Koszinowski, Ulrich H., 80805 München (DE)
(72) Erfinder: Wagner, Markus Dr., 80469 München (DE); Brune, Wolfram Dr., Princeton, NJ 08540 (US)
(74) Vertreter: Albrecht, Thomas, Dr.

(57) **Zusammenfassung**

Die Erfindung betrifft ein Vektorsystem zum Einbringen von DNA in eukaryonte Zellen, das Vektorsystem umfaßt Bakterien, die von eukaryonten Zellen aufgenommen werden oder die aktiv in eukaryonte Zellen eindringen können (invasive Bakterien), wobei die Bakterien ein eine heterologe DNA enthaltendes bakterielles künstliches Chromosom (BAC) umfassen. Bei der heterologen DNA handelt es sich vorzugsweise um die DNA (oder Teile davon) eines DNA-Virus. Die virale DNA kann zusätzlich weitere heterologe Gene enthalten. In Abhängigkeit der Natur der weiteren heterologen Gene und in Abhängigkeit der Natur der viralen DNA kann das Vektorsystem unter anderem als Impfstoff oder Arzneimittel verwendet werden.

## Beschreibung

Die Erfindung betrifft ein Vektorsystem zum Einbringen von DNA in eukaryonte Zellen, umfassend invasive Bakterien, die ein heterologe DNA enthaltendes bakterielles künstliches Chromosom ("BAC") enthalten. Bei der heterologen DNA kann es sich insbesondere um die DNA eines DNA-Virus oder um eine DNA-Kopie der RNA eines RNA-Virus handeln. Diese virale DNA kann zusätzlich weitere heterologe Gene enthalten. Das Vektorsystem, das invasive Bakterien und ein bakterielles künstliches Chromosom enthält, kann insbesondere als Impfstoff oder Arzneimittel verwendet werden. Die Erfindung umfaßt weiterhin ein Verfahren zur Charakterisierung von Virusmutanten in bakteriellen künstlichen Chromosomen unter Verwendung von invasiven Bakterien. Die Erfindung betrifft weiterhin ein Verfahren zum ortsspezifischen Einfügen eines heterologen DNA-Abschnittes in Virus-DNA enthaltende bakterielle künstliche Chromosomen.

Der wirksamste Schutz vor den Folgen einer mikrobiellen oder viralen Infektion ist die Impfung. Die Entwicklung wirksamer Impfstoffe ist bisher aber nur für einen kleinen Teil der human- und tierpathogenen Bakterien und Viren gelungen. Lebendimpfstoffe, die meist effizienter in ihrer Wirkung sind, da sie aktiv die immunogenen Antigene vermehren, sind aufwendig zu produzieren. So müssen zum Beispiel für die Herstellung von viralen Lebendimpfstoffen diese Viren auf eukaryontischen Zellen angezüchtet und anschließend zur Aufbewahrung und für den Transport kühl gelagert werden, um Inaktivierung zu vermeiden.

Es wurde gezeigt, daß eine Impfung grundsätzlich auch dadurch möglich ist, daß Antigen kodierende DNA durch intramuskuläre Injektion verabreicht wird (siehe z.B. Tascon et al., Nat. Med. (1996), 2, 888-892). Bei derartigen DNA-Vaccinierungsversuchen werden in der Regel übliche E. coli-Plasmide verwendet. Die Klonierungskapazität dieser Plasmide ist beschränkt. Es ist beispielsweise nicht möglich, die Virus-DNA großer DNA-Viren, wie z.B. Herpesviren, vollständig in derartige bakterielle Plasmide zu klonieren. Für bestimmte therapeutische Anwendungen ist es jedoch von großem Interesse, große DNA-Stücke in Plasmiden zu klonieren und diese in eukaryonte Zellen einzubringen. Insbesondere kann es wünschenswert sein, replikationskompetente virale Genome, die gegebenenfalls weitere heterologe Gene enthalten, in Plasmide zu klonieren. Das Einbringen derartiger Plasmidkonstrukte in eukaryonte Zellen kann dazu führen, daß die in den Plasmiden enthaltene Virus-DNA in den Zellen repliziert. Im Zuge dieser Replikation könnte auch die zusätzliche heterologe DNA in der viralen DNA, bei der es sich beispielsweise um ein therapeutisches oder immunogenes Protein handelt, vermehrt werden. Eine solche Vermehrung eines immunogenen Proteins ist beispielsweise für eine effiziente Präsentation dieses Proteins gegenüber Immun-Effektorzellen und damit einer wiksamen Immunantwort notwendig.

Da das Klonieren sehr großer DNA-Fragmente mit üblichen E. coli-Plasmiden nicht möglich ist, wurden in der Vergangenheit künstliche bakterielle Chromosomen für derartige Zwecke verwendet (z.B. pBeloBAC11 oder pBAC108L, siehe z.B. Shizuya et al., Proc. Natl. Acad. Sci. USA (1992), 89, 8794-8797; Wang et al., Biotechniques (1997), 23, 992-994). Die WO 99/43842 beschreibt die Klonierung des vollständigen Herpes simplex-Virusgenomes in ein derartiges bakterielles artifizielles Chromosom. Die WO 99/43842 beschreibt weiter, daß die Virus-DNA weitere heterologe Gene enthalten kann, die beispielsweise für ein therapeutisches Gen-Produkt, wie z.B. einen Wachstumsfaktor, ein Hormon, ein Impfantigen, eine Antisense-RNA oder ein Ribozym kodieren. Die WO 99/43842 beschreibt weiterhin die Verwendung derartiger künstlicher Chromosomenkonstrukte, um gegen ein Virus zu impfen, dessen DNA in einem derartigen Konstrukt enthalten ist. Um solche Konstrukte in eukaryonte Zellen einzubringen, beschreibt die WO 99/43842 verschiedene Wege, wie z.B. direkte Injektionen, gegebenenfalls unter Verwendung chirurgischer Verfahren, intravenöse, subkutane, intradermale, intraepidermale oder intramuskuläre Verabreichungswege. Weiterhin wird beschrieben, daß derartige BAC-Konstrukte auch in Liposomen eingebracht werden können oder mittels kationischer Lipide, Gold- oder Wolfram-Mikropartikel verabreicht werden können. Die vorgeschlagenen Verfahren sind jedoch für eukaryonte Zellen, insbesondere für vollständige Organismen nur wenig effizient. Dadurch kann eine Impfung von Menschen oder Tieren nur mit geringer Effizienz erfolgen.

Auf der anderen Seite wurde beschrieben, daß ein funktioneller Gentransfer mittels invasiver Bakterien stattfinden kann. Bei invasiven Bakterien handelt es sich um Bakterien, die von eukaryonten Zellen aufgenommen werden können oder die aktiv in eukaryonte Zellen eingebracht werden können. Enthalten invasive Bakterien heterologe DNA, beispielsweise Plasmid-DNA, so wird diese heterologe DNA zusammen mit dem invasiven Bakterium von den eukaryonten Zellen aufgenommen und es kann zur Expression der heterologen Gene kommen. Ein derartiges Einbringen heterologer DNA in eukaryonte Zellen wurde z.B. unter Verwendung von Salmonella typhimurium (Darji et al., Cell (1997) 91, 765-775), Shigella (Sizemore et al., Science (1995), 270, 299-302) oder invasiven Escherichia coli-Varianten (Grillot-Courvalin et al., Nature Biotechnology (1998), 16, 862-866) beschrieben. In diesen Arbeiten konnte zum Teil ein funktioneller Gentransfer von den invasiven Bakterien in Säugetierzellen beschrieben werden. Allerdings handelte es sich bei den in diesen Arbeiten beschriebenen Plasmiden, die von den invasiven Bakterien in die eukaryonten Zellen eingebracht wurden, nur um kleinere high-copy-Plasmide, in keinem Fall um Plasmide, mit denen beispielsweise das Genom großer DNA- oder RNA-Viren in die Zellen eingebracht wurde.

Der Erfindung lag somit die Aufgabe zugrunde, ein Vektorsystem zu entwickeln, das es ermöglicht, mit hoher Effizienz, große heterologe DNA-Moleküle - beispielsweise virale Genome, insbesondere rekombinante virale Genome, die weitere/zusätzliche DNA-Abschnitte enthalten, die für therapeutische oder immunogene Genprodukte codieren - in eukaryonte Zellen einzubringen. Dabei sollten insbesondere auch die Nachteile der bekannten Verabreichungsverfahren, insbesondere von Verabreichungsverfahren, die in jedem Fall eine Injektion notwendig machen, vermieden werden können. Weiterhin war es eine Aufgabe der Erfindung, das Vektorsystem dergestalt zu entwickeln, daß beispielsweise zum Zwecke einer Immunisierung ein einfaches Schlucken des Vektorsystems ausreichend ist, um einen entsprechenden Erfolg zu erzielen.

Diese Aufgabe wurde durch das Bereitstellen eines Vektorsystems zum Einbringen von DNA in eukaryonte Zellen gelöst, das Bakterien umfaßt, die von eukaryonten Zellen aufgenommen werden können oder aktiv in die eukaryonte Zelle eindringen können ("invasive Bakterien"), wobei die Bakterien ein eine heterologe DNA enthaltendes bakterielles künstliches Chromosom ("BAC") umfassen. Durch die Verwendung von invasiven Bakterien ist es möglich, heterologe DNA einfach durch Inkontaktbringen der invasiven Bakterien mit eukaryonten Zellen in die letzteren einzubringen. Handelt es sich bei den eukaryonten Zellen um einen Gesamtorganismus, wie z.B. einen Menschen oder ein Tier, kann das Vektorsystem falls notwendig oder gewünscht einfach durch Schluckimpfung verabreicht werden.

Durch die Verwendung von bakteriellen künstlichen Chromosomen ist es möglich, sehr große heterologe DNA über die invasiven Bakterien in die eukaryonten Zellen einzubringen.

Bei "invasiven Bakterien" handelt es sich um Bakterien, die in der Lage sind, von eukaryonten Zellen aufgenommen zu werden oder um Bakterien, die aktiv in eukaryonte Zellen eindringen. Insbesondere wird unter dem Begriff "Invasion" verstanden, daß ein Bakterium in der Lage ist, in eine eukaryonte Zelle einzudringen und das Cytoplasma durch Lyse der Vakuolen-Membranen zu erreichen. Im Gegensatz dazu sind nicht-invasive Bakterien Bakterien, die nicht oder zumindest nur in vernachlässigbar geringem Ausmaß von eukaryonten Zellen aufgenommen werden können und die i.d.R. nicht aktiv in eukaryonte Zellen eingebracht werden können. Bei einer besonders bevorzugten Ausführungsform sind die invasiven Bakterien aktiv in der Lage, in eukaryonte Zellen einzudringen. Darji et al., Cell (1997), 91, 765-775 beschreiben orale somatische Transgen-Vaccinierung unter Verwendung attenuierter invasiver Salmonella typhimurium. Sizemore et al., Science (1995), 270, 299-302 beschreiben die Verwendung von Shigella, das in der Lage ist, in epitheliale Zellen einzudringen, wobei es der phagocytotischen Vakuole entkommt. Shigella wird dazu verwendet, um Plasmid-DNA in das Cytoplasma der Wirtszelle einzubringen und um Protein zu synthetisieren, das zur Antigenpräsentation prozessiert wird. Weitere invasive Bakterien sind beispielsweise auch Stämme der Gattungen Yersinia und Listeria. Der Transfer von Vektoren in Makrophagen durch Lysteria monocytogenes wurde beispielsweise von Dietrich et al., Nature Biotechnology (1998), 16, 181-185 beschrieben. Gentransfer unter Verwendung von Escherichia coli wurde von Schaffner 1980 beschrieben (Proc. Natl. Acad. Sci. USA, 77, 2163-2167). Im Zusammenhang mit der vorliegenden Erfindung betrifft der Begriff "invasive Bakterien" somit alle Bakterien, die in der Lage sind, in eukaryonte Zellen einzudringen oder von diesen aufgenommen zu werden. Insbesondere betrifft die Erfindung invasive Bakterien, in denen das wie unten definierte inv-Gen und gegebenenfalls das hly-Gen exprimiert wird.

Die Erfindung ist nicht beschränkt auf die natürlicherweise invasiven Bakterienstämme der Gattungen Yersinia (insbesondere Y. pseudotuberculosis und Y. enterocolitica), Shigella, Salmonella und Escherichia. Bei den invasiven Bakterien kann es sich auch um Bakterien handeln, die natürlicherweise nicht-invasiv sind, jedoch ein oder mehrere Fremdgene enthalten können, die dazu führen, daß das natürlicherweise nicht-invasive Bakterium zu einem invasiven Bakterium wird.

Bei einem derartigen natürlicherweise nicht-invasiven Bakterienstamm kann es sich beispielsweise um einen nicht-invasiven Salmonella typhimurium- oder Escherichia coli-Stamm handeln. Verfahren, um natürlicherweise nicht-invasive Baktieren in invasive Bakterien umzuwandeln, indem geeignete Fremdgene in die natürlicherweise nicht-invasiven Bakterien eingebracht und in diesen exprimiert werden, sind dem Fachmann bekannt (Grillot-Courvalin et al., Nature Biotechnology (1998), 16, 862-866 und FR 2 743 086).

Insbesondere kann ein nicht-invasives Bakterium (oder ein gegebenenfalls nur in geringem Maße invasives Bakterium) dadurch invasiv gemacht werden, daß das Invasin-Gen von Yersinia pseudotuberculosis oder Yersinia enterocolitica ("inv-Gen") eingebracht (Grillot-Courvalin et al., Nature Biotechnology (1998), 16, 862-866) und das entsprechende inv-Protein gebildet wird. Um eine Freisetzung des aufgenommenen Bakteriums aus den Vesikeln zu erleichtern, wird in das natürlicherweise nicht-invasive Bakterium vorzugsweise noch das Listeriolysin-O-Gen (hly-Gen) von Listeria monocytogenes eingeführt und exprimiert (Grillot-Courvalin et al., Nature Biotechnology (1998), 16, 862-866). Das inv-Gen und gegebenenfalls das hly-Gen kann mittels dem Fachmann bekannter Techniken in das natürlicherweise nicht-invasive Bakterium eingebracht werden. Vorzugsweise sind in den natürlicherweise nicht-invasiven Bakterien sowohl das inv- als auch das hly-Gen enthalten, am meisten bevorzugt gemeinsam auf einem Plasmid oder im bakteriellen Genom verankert. In jedem Fall muß das inv-Gen und gegebenenfalls das hly-Gen in den Zellen exprimiert werden können. Geeignete konstitutive oder induzierbare Promotoren sind dem Fachmann bekannt. Ein Beispiel für ein Plasmid, das das inv-Gen und das hly-Gen exprimiert, ist das Plasmid pGBΩinv-hly (Grillot-Courvalin et al., Nature Biotechnology (1998), 16, 862-866).

Das inv- und gegebenenfalls das hly-Gen kann auch dazu verwendet werden, um eine geringe Invasionsaktivität bestimmter Bakterienstämme zu erhöhen.

Um die Sicherheit eines derartigen Vektorsystems auf Basis von invasiven Bakterien zu erhöhen, kann es sich bei den invasiven Bakterien - unabhängig davon, ob es sich um natürlicherweise invasive Baktieren handelt oder um Bakterien, die natürlicherweise nicht-invasiv sind, jedoch mit den entsprechenden Genen transfiziert wurden - um Bakterien handeln, die in eukaryonten Zellen nicht überleben können oder sich zumindest in diesen nicht vermehren können. Dem Fachmann ist bekannt, wie er dies erreichen kann. So beschreibt die FR 2 743 086 z.B. E. coli-Stämme, die dadurch in bezug auf die Diaminopimelinsäure auxotroph sind. Diaminopimelinsäure ist eine Verbindung, die für die Synthese der bakteriellen Zellwand notwendig ist. Insbesondere wird vorgeschlagen, das dapA-Gen von E. coli zu inaktivieren. Dieses Gen kodiert für die Dihydrodipicolinat-Synthetase. Dieses Enzym ist notwendig für die Synthese von Diaminopimelinsäure. Da eine entsprechende Aktivität in eukaryonten Zellen nicht bekannt ist, führt somit der Ausfall dieser enzymatischen Aktivität zum Verlust der Fähigkeit von E. coli-Zellen Diaminopimelinsäure zu bilden und somit eine bakterielle Zellwand aufzubauen.

Unter dem Begriff "eukaryonte Zellen" im Zusammenhang mit der vorliegenden Erfindung werden jegliche eukaryonte Zellen, unabhängig davon, ob sie sich in Zell- oder Gewebekultur befinden oder ob sie einen eukaryonten Gesamtorganismus bilden, verstanden. In diesem Zusammenhang umfaßt der Begriff eukaryonte Zellen - wenn nicht anders definiert - auch einen tierischen oder menschlichen Gesamtorganismus, so daß das erfindungsgemäße Vektorsystem auch zum Einbringen von DNA in Tiere oder Menschen geeignet ist.

Unter dem Begriff "bakterielle künstliche Chromosomen" oder "artifizielle bakterielle Chromosomen", abgekürzt als "BAC" werden große Vektoren auf DNA-Basis verstanden. Diese Vektoren beinhalten Elemente, die von bakteriellen F-Plasmiden abgeleitet sind und die für die Replikation und die Aufrechterhaltung/Persistenz in den Zellen verantwortlich sind. In bakterielle artifizelle Chromosomen können große genomische DNA-Fragemente kloniert werden. Die so erhaltenen bakteriellen künstlichen Chromosomen mit heterologer DNA können in prokaryonte Zellen eingebracht werden, wo sie stabil in kontrollierter Kopienzahl persistieren. Auf diese Art und Weise ist es somit auch möglich, große heterologe DNA-Fragmente in prokaryonte Zellen zu klonieren. Beispiele bakterieller künstlicher Chromosomen sind dem Fachmann bekannt. Beispielsweise wird hier auf die oben bereits erwähnten Konstrukte pBeloBAC11 oder pBAC108L verwiesen (Shizuya et al., Proc. Natl. Acad. Sci. USA (1992), 89, 8794-8797 und Wang et al., Biotechiques (1997), 23, 992-994) .

Erfindungsgemäß enthält das bakterielle künstliche Chromosom heterologe DNA. Unter dem Begriff "heterologe DNA" in bezug auf bakterielle künstliche Chromosomen wird DNA verstanden, die in dem bakteriellen künstlichen Chromosom ursprünglich nicht enthalten ist. In bezug auf die oben erwähnten Konstrukte pBeloBAC11 oder pBAC108L wird somit unter heterologer DNA jegliche DNA verstanden, die in diesen Ausgangskonstrukten ursprünglicherweise nicht enthalten ist. Bei der heterologen DNA kann es sich um beliebige prokaryonte, eukaryonte oder insbesondere virale DNA handeln. Vorzugweise ist die in dem BAC enthaltene heterologe DNA virale DNA. Die virale DNA kann dabei von einem DNA-Virus stammen.

Beispiele für DNA-Viren sind große DNA-Viren, wie z.B. die humanen Herpesviren 1-8, sowie die animalen Herpesviren Pseudorabiesvirus, Marek disease virus, murines Cytomegalovirus, murines γ-Herpesvirus 68, Herpesvirus saimiri, oder Adenovirus-DNA. Bei der viralen DNA kann es sich auch um die DNA-Kopie der RNA eines RNA-Virus handeln. Beispiele für RNA-Viren sind Picornaviren (z.B. Polio-, Hepatitis A-Virus), Flaviviren (z.B. Hepatitis C-Virus), Paramyxoviren (Mumps-, Masernvirus), Orthomyxoviren (z.B. Influenza) und Rhabdoviren (Rabiesvirus).

Verfahren zur Herstellung einer DNA-Kopie eines RNA-Virus sind dem Fachmann geläufig.

Bevorzugt handelt es sich bei der heterologen viralen DNA um die DNA eines Herpesvirus. Am meisten bevorzugt handelt es sich bei der DNA eines Herpesvirus um die DNA eines Cytomegalovirus (CMV-Virus). Bei der CMV-Virus-DNA handelt es sich bevorzugterweise um das humane Cytomegalovirus, am meisten bevorzugt um das murine Cytomegalovirus.

Dem Fachmann sind Verfahren bekannt, mit denen er heterologe DNA, insbesondere große virale DNA-Fragmente und insbesondere vollständig virale Genome in ein bakterielles artifizielles Chromosom klonieren kann. Insbesondere sind dem Fachmann auch Verfahren bekannt, mit denen er die DNA großer Herpesviren in bakterielle artifizelle Chromosomen klonieren kann. Beispielsweise können BACs, die Virus-DNA enthalten, dadurch hergestellt werden, daß die Virus-DNA oder Fragmente davon direkt in die BACs kloniert wird. Alternativ ist es auch möglich, die Virus-DNA mittels homologer Rekombination in die bakteriellen Chromosomen zu klonieren. Soll das künstliche bakterielle Chromosomenkonstrukt umfangreiche virale Sequenzen enthalten, die es ermöglichen, daß ein replikationskompetentes und infektiöses Virus in den Zellen entsteht, so sollte eine homologe Rekombination so vorgenommen werden, daß bei dieser homologen Rekombination lediglich ein nicht essentieller Bereich des Virusgenoms beteiligt ist. Im Falle von Herpes simplex-Virus kann eine Rekombination im Bereich des Thymidinkinase-Bereichs erfolgen (siehe z.B. WO 99/43842). Verfahren zur Herstellung von rekombinanten bakteriellen artifiziellen Chromosomen, die infektiöse virale Genomsequenzen mit einer Größe von mehr als 100 kb enthalten, sind beispielsweise in PCT/EP98/04816, WO 99/43842 und in Messerle et al., Proc. Natl. Acad. Sci. USA (1997), 94, 14759-14763 beschrieben.

Gemäß der am meisten bevorzugten Ausführungsform betrifft die Erfindung ein Vektorsystem, umfassend invasive Bakterien, in denen das wie oben definierte inv-Gen und gegebenenfalls das hly-Gen exprimiert wird und die ein virale DNA enthaltendes BAC enthalten. Die virale DNA ist in dieser bevorzugten Ausführungsform insbesondere die virale DNA (bzw. Teile davon) großer DNA-Viren, mehr bevorzugt Herpesviren, am meisten bevorzugt Cytomegaloviren. Die virale DNA kann zusätzlich einen wie unten definierten weiteren/zusätzlichen DNA-Abschnitt enthalten.

Bei der Virus-DNA kann es sich um nicht rekombinante, natürlich vorkommende Virus-DNA handeln. In einer bevorzugten Ausführungsform ist die virale DNA infektiös und/oder replikationskompetent. In diesem Fall müssen in der Virus-DNA im bakteriellen künstlichen Chromosom alle Gene funktionell enthalten sein, die für die Replikation notwendig sind und die notwendig sind damit infektiöse Viruspartikel gebildet werden.

In einer weiteren bevorzugten Ausführungsform ist die Virus-DNA nicht infektiös und/oder replikationskompetent. In diesem Fall ist mindestens ein Gen, dessen Genprodukt für die Replikationsfähigkeit und/oder die Infektiosität notwendig ist, nicht enthalten oder inaktiviert. Bei einer weiteren bevorzugten Ausführungsform der Erfindung umfaßt die virale DNA nur Teile der vollständigen, natürlicherweise vorkommenden Virus-DNA.

Um die Freisetzung von viraler DNA aus bakteriellen Chromosomen zu ermöglichen, kann die Virus-DNA in den BACs vorzugsweise von identischen Sequenzabschnitten flankiert sein. In diesem Fall ist es möglich, daß über die identischen Abschnitte eine homologe Rekombination stattfindet, die zur Freisetzung der Virus-DNA aus dem BAC führt.

Vorzugsweise kann die Virus-DNA zusätzlich weitere DNA-Abschnitte viralen oder nicht viralen Ursprungs enthalten. Verfahren zum Einbringen von zusätzlichen DNA-Abschnitten in die virale DNA sind dem Fachmann bekannt. Eine bevorzugte Ausführungsform zum Einbringen zusätzlicher DNA-Abschnitte unter Verwendung von PCR-Fragmenten wird weiter unten beschrieben. Grundsätzlich gibt es zur Herstellung der entsprechenden bakteriellen artifiziellen Chromosomen zwei Herstellungswege. Auf dem ersten Herstellungsweg kann zunächst mittels dem Fachmann bekannter Verfahren eine gewünschte Virus-DNA in das bakterielle artifizielle Chromosom einkloniert werden und in einem zweiten Verfahrensschritt kann nun zusätzliche DNA spezifisch in den viralen DNA-Abschnitt im bakteriellen artifiziellen Chromosom einkloniert werden. Alternativ kann der zusätzliche DNA-Abschnitt zunächst in die Virus-DNA kloniert werden und die entsprechende rekombinante Virus-DNA kann dann mittels dem Fachmann bekannter Verfahren in das bakterielle artifizielle Chromosom kloniert werden (siehe z.B. Messerle et al., Proc. Natl. Acad. Sci. USA (1997), 94, 14759-14763).

Soll die in den BACs enthaltene virale DNA infektiös und replikationskompetent sein, so ist es notwendig, daß die zusätzlichen DNA-Abschnitte, die in die virale DNA eingeführt werden, zusammen mit der viralen DNA nicht die Gesamt-DNA-Größe überschreiten, die maximal in Viruspartikel verpackt werden kann.

Erfindungsgemäß kann der weitere/zusätzliche DNA-Abschnitt in der viralen DNA für ein oder mehrere zusätzliche Genprodukte codieren. Bei einem derartigen zusätzlichen Genprodukt kann es sich beispielsweise um ein Markergen oder einen Selektionsmarker handeln. Beispiele für derartige Selektions- oder Markergene wurden bereits im Zusammenhang mit den Selektions- und Markergenen des bakteriellen artifiziellen Chromosoms aufgezeichnet. Vorzugsweise ist das Selektions- oder Markergen, das in der Virus-DNA enthalten ist, von dem Selektions- oder Markergen verschieden, das im bakteriellen artifiziellen Chromosom enthalten ist. Somit ist es möglich, mittels eines ersten Selektionsmarkers auf das Vorhandensein eines bakteriellen artifiziellen Chromosoms im allgemeinen und mit einem zweiten Selektions- oder Markergen auf das Vorhandensein von Virus-DNA im bakteriellen artifiziellen Chromosom zu selektionieren.

Bei dem weiteren/zusätzlichen DNA-Abschnitt in der viralen DNA kann es sich auch um DNA handeln, die für ein oder mehrere therapeutische und/oder immunogene Genprodukte kodiert. Beispiele für derartige Genprodukte sind Wachstumsfaktoren, Hormone, Enzyme, Impfantigene, Cytokine, immunomodulatorische Proteine, Antisense-RNA-Moleküle und Ribozyme. Von der Erfindung mitumfaßt sind Gene, die für funktionelle Derivate der oben genannten Genprodukte kodieren. Bei funktionellen Derivaten handelt es sich um Genprodukte, die im wesentlichen die gleiche Funktion haben wie das natürlicherweise vorkommende Genprodukt, deren Aminosäuresequenz bzw. RNA-Sequenz allerdings von den natürlicherweise vorkommenden Sequenzen abweicht. So ist beispielsweise ein funktionelles Derivat eines Wachstumsfaktors, eines Hormons, eine Enzymes, Impfantigens, Cytotoxins oder eines immunomodulatorischen Proteins ein Peptid/Protein mit einer von der natürlicherweise vorkommenden Sequenz abweichenden Aminosäuresequenz, wobei das Peptid/Protein mit der abweichenden Proteinsequenz im wesentlichen die gleiche Funktion hat wie das natürlicherweise vorkommende Peptid/Protein. Bei Proteinen/Peptiden kann es sich hierbei insbesondere um Peptide/Proteine handeln, die eine oder mehrere Deletionen, Substitutionen oder Insertionen aufweisen. Funktionelle Derivate von Peptiden/Proteinen sind insbesondere Peptide/Proteine, die die gleiche Funktion wie das natürlicherweise vorkommende Peptid/Protein haben und die eine mindestens 60%ige, bevorzugterweise mindestens 80%ige, am meisten bevorzugt mindestens 90%ige Homologie auf Aminosäureebene besitzen.

Vorzugsweise stellt der DNA-Abschnitt, der für ein weiteres/zusätzliches Genprodukt kodiert, eine funktionelle Expressionskassette dar. Eine derartige Expressionskassette umfaßt regulatorische Elemente, insbesondere Promotoren/Enhancer und Terminations-/Translationskontrollelemente. Dem Fachmann sind derartige Elemente bekannt. Beispielsweise kann ein zelltypspezifischer oder tumorspezifischer Promotor verwendet werden, um die Expression des weiteren/zusätzlichen Genprodukts auf einen bestimmten Zelltyp zu beschränken, was insbesondere dann vonnutzen ist, wenn beispielsweise ein cytotoxisches oder immodulatorisches oder aber ein Tumorantigen-Genprodukt in einer Tumorzelle gebildet werden soll, um ihre Zerstörung zu erleichtern. Um eine Expression des zusätzlichen Genprodukts in einer Vielzahl von Zellen zu erreichen, können nicht gewebsspezifische Promotoren verwendet werden. Beispiele für spezifische und nicht spezfifische Promotoren sind dem Fachmann bekannt (siehe z.B. WO 99/43842).

Der weitere/zusätzliche DNA-Abschnitt im Virusgenom kann erfindungsgemäß auch ohne eigenen Promotor/Enhancer in der Virus-DNA enthalten sein. In diesem Fall ist der zusätzliche DNA-Abschnitt vorzugsweise im gleichen Leseraster in ein virales Gen integriert oder aber der zusätzliche DNA-Abschnitt liegt in Fusion mit einem viralen Gen vor. Vorzugsweise kodiert der weitere/zusätzliche DNA-Abschnitt im viralen Genom in diesem Fall für ein immunogenes Epitop. Unter dem Begriff "immunogenes Epitop" wird jede Aminosäuresequenz verstanden, die in der Lage ist, in einem Wirtsorganismus eine Immunantwort zu induzieren. Bei einer derartigen Immunantwort kann es sich um eine humorale Immunantwort oder aber um eine zelluläre Immunantwort handeln. Beispiele für immunogene Epitope sind z.B. Epitope aus dem HBs-Antigen, dem Tetanus-Toxin und dem Diphtheria-Toxin.

Sollen heterologe Gene im Kontext viraler DNA in einem BAC enthalten sein, so ist die DNA großer viraler DNA-Vektoren (insbesondere von Herpes-viralen Vektoren) besonders geeignet. Der Vorteil derartiger großer DNA-Vektoren liegt unter anderem in der großen Kapazität für Fremdgeninsertionen, da große Teile des Genoms für die Replikation und Verpackung nicht essentiell sind und dadurch nach der Deletion derartiger Genomteile Platz für therapeutische oder Antitumorgene bieten. Besonders bevorzugt ist das Cytomegalovirus, da es das größte DNA-Genom unter diesen Viren besitzt und große Bereiche durch Fremd-DNA ersetzt werden können. Enthält das erfindungsgemäße Vektorsystem somit bakterielle künstliche Chromosomen, die eine derartige Virus-DNA/Fremd-DNA enthalten und ist der in den bakteriellen künstlichen Chromosomen enthaltene virale Teil noch replikationsfähig und infektiös, so werden nach Einbringen des erfindungsgemäßen Vektorsystems in eukaryonte Zellen neue Viren gebildet, die durch Zell-zu-Zellverbreitung eine starke Virusvermehrung ermöglichen. Die auf jedem Virusgenom gegebenenfalls vorhandenen therapeutisch oder immunogen aktiven Gene werden dadurch zusätzlich vermehrt und die erzielte Wirkung der resultierenden Genprodukte wird verstärkt. Die vorliegende Erfindung beschreibt somit zum erstenmal die Möglichkeit, ein bakterielles künstliches Chromosom mit einer Genomgröße von mindestens bis zu 240 kbp, das in einem intakten, invasiven Bakterium vorliegt, direkt und ohne DNA-Isolierung oder Anwendung von Transfektionsmethoden durch einfaches Zugeben zu eukaryonten Zellen in diese zu überführen.

Erfindungsgemäß befinden sich bei einer bevorzugten Ausführungsform zwischen der BAC-DNA und der einklonierten heterologen DNA Erkennungssequenzen für sequenzspezifische Rekombinasen und/oder für Restriktionsendonukleasen, die sich von den Sequenzen des restlichen Vektors unterscheiden. Befinden sich derartige Sequenzen zwischen der BAC-DNA und den einklonierten heterologen DNAs, so können letztere herausgeschnitten werden, ohne daß das Grundgerüst des BAC als solches zerstört wird. Vorzugsweise handelt es sich bei den Erkennungssequenzen für sequenzspezifische Rekombinasen um loxP-Stellen (siehe z.B. PCT/EP98/0816) oder FRT-Stellen (FRT = FLP recombination target; siehe McLeod et al., Mol. Cell. Biol. (1986), 6, 3357-3367).

Das erfindungsgemäße Vektorsystem wird dadurch hergestellt, daß das gewünschte artifizielle bakterielle Chromosom mit der gewünschen heterologen DNA in einem invasiven oder nicht-invasiven Bakterium (vorzugsweise einem nicht-invasiven E. coli-Stamm) hergestellt wird. Ist das Bakterium nicht-invasiv, so wird in einem zweiten Schritt in das Bakterium - das das so hergestellte BAC-Konstrukt, welches heterologe DNA (beispielsweise Virus-DNA mit zusätzlichen therapeutischen Genen) enthält - ein Plasmid eingebracht, das Gene enthält, die das Bakterium invasiv machen (inv- und hly-Gen). Bei einem alternativen zweiten Schritt kann das BAC-Konstrukt nach der Herstellung in einem nicht-invasiven Bakterium isoliert und in ein invasives Bakterium eingebracht werden. Verfahren zum Einbringen von Plasmiden oder BAC-DNA in Bakterien sind dem Fachmann bekannt. Derartige Verfahren umfassen beispielsweise Standardtransfektionsansätze mit Calciumchlorid oder Elektroporation.

Die Erfindung umfaßt weiterhin ein Verfahren zum Einbringen von DNA in eukaryonte Zellen. Dieses Verfahren ist dadurch gekennzeichnet, daß die eukaryonten Zellen mit einem wie oben beschriebenen Vektorsystem, das invasive Bakterien und ein heterologe DNA enthaltendes artifizielles Chromosom umfaßt, in Kontakt gebracht werden. Handelt es sich bei den eukaryonten Zellen um eine Zell- oder Gewebekultur, so kann eine entsprechende Suspension des erfindungsgemäßen Vektorsystems dem Zellkulturmedium zugegeben werden. Handelt es sich bei dem eukaryonten Organismus um einen Gesamtorganismus, so können die weiter unten beschriebenen Verabreichungswege verwendet werden.

Wird das erfindungsgemäße Vektorsystem, umfassend invasive Baktieren, die ein eine heterologe DNA enthaltendes bakterielles künstliches Chromosom umfassen, eukaryonten Zellen verabreicht, so werden die invasiven Bakterien von eukaryonten Zellen aufgenommen. In den eukaryonten Zellen kommt es dann zur Expression der im bakteriellen künstlichen Chromosom enthaltenen heterologen DNA soweit diese eine eukaryonte Expressionskassette enthält. Handelt es sich bei den eukaryonten Zellen um einen Gesamtorganismus, wie z.B. den Menschen oder ein Tier, wird somit die heterologe DNA in Menschen oder Tieren exprimiert. Handelt es sich bei der heterologen DNA um ein therapeutisches Gen, so führt dies zur Bildung des therapeutischen Genprodukts. Handelt es sich bei der heterologen DNA um eine Virus-DNA, so sind mehrere Fälle zu unterscheiden:

Ist die Virus-DNA nicht mehr replikationskompetent oder infektiös, enthält jedoch noch funktionelle virale Gene, so werden lediglich virale Genprodukte gebildet, ohne daß in den Zellen selbst das Virus gebildet wird und/oder sich vermehrt. Sind die noch gebildeten viralen Genprodukte immunogen, so können, insbesondere dann, wenn es sich bei den eukaryonten Zellen um Antigen präsentierende Zellen handelt, diese an der Zelloberfläche präsentiert werden. Es kann somit die Entwicklung einer Immunantwort gegen derartige virale Antigene induziert werden. Bei dieser bevorzugten Ausführungsform ist somit eine Impfung gegen das Virus möglich, von dem die heterologe DNA, die im bakteriellen künstlichen Genom enthalten ist, stammt. Bei einer weiteren bevorzugten Ausführungsform ist die Virus-DNA noch replikationskompetent, aber nicht mehr infektiös. Im Zusammenhang mit der vorliegenden Anmeldung wird darunter verstanden, daß die Virus-DNA in den eukaryonten Zellen zwar noch vermehrt werden kann, jedoch keine Viruspartikel mehr gebildet werden. Bei einer besonders bevorzugten Ausführungsform bildet die replikationskompetente aber nicht infektiöse Virus-DNA keinerlei virale Genprodukte mehr, die an der Zelloberfläche exprimiert werden können. Enthält die virale DNA in diesem Fall ein weiteres/zusätzliches heterologes Gen, das beispielsweise für ein immunogenes oder ein therapeutisches Gen kodiert, so kommt es in den Zellen zur Expression derartiger therapeutischer/immunogener Genprodukte, ohne daß jedoch eine Immunantwort gegen das Virus selbst induziert werden kann, da das Virus derartige, für das Virus spezifische Epitope nicht an der Oberfläche der Antigen präsentierenden Zellen exprimiert. Derartige Vektorsysteme, also Vektorsysteme, die invasive Bakterien mit einem BAC umfassen, wobei das BAC replikationskompetente, aber nicht infektiöse Virus-DNA mit einem therapeutischen Gen enthält, können beispielsweise für gentherapeutische Zwecke verwendet werden. Dadurch, daß keine viralen Epitope an der Oberfläche der Zelle exprimiert werden, wird gegen die Virus-DNA enthaltenden Zellen somit keine spezifische Immunantwort aufgebaut. Dies ist eine Voraussetzung dafür, daß ein Vektorsystem mehrfach verabreicht werden kann.

Ist die Virus-DNA noch replikationskompetent und infektiös, so kommt es nach dem Einbringen des erfindungsgemäßen Vektorsystems in die eukaryonte Zelle bzw. in den Gesamtorganismus zur Replikation der Virus-DNA und zur Freisetzung infektiöser Viren. Vorzugsweise sind die infektiösen Viren nicht pathogen. Bei den nicht pathogenen Viren handelt es sich entweder um natürlicherweise apathogene Viren oder um Viren, die eine Virus-DNA enthalten, in der Gene inaktiviert wurden, die für die Pathogenität verantwortlich sind. Derartige apathogene Viren werden auch als attenuierte Viren bezeichnet. Dadurch, daß die Virus-DNA replikationskompetent und infektiös ist, können somit nach dem Einbringen des Vektorsystems in die eukaryonte Zelle große Mengen an Nachkommenviren gebildet werden, die unter Umständen in der Lage sein können, andere eukaryonte Zellen im Gesamtorganismus zu infizieren. Dies fördert in starker Weise die Immunantwort des Organismus. Besonders bevorzugt sind zum Zweck der Immunisierung BACs, die Virus-DNA enthalten, wobei die Virus-DNA zusätzlich geeignete Adjuvantien oder immunstimulierende Genprodukte, wie z.B. Cytokine, exprimiert. Die Expression der zusätzlichen Adjuvantien oder immunstimulierenden Gene führt dazu, daß die Immunantwort gegen das Virus noch weiter verstärkt wird. Ist der zusätzliche DNA-Abschnitt in der Virus-DNA ein immunogenes Epitop, so kann dieser DNA-Abschnitt auch im gleichen Leserahmen in ein virales Gen integriert sein oder in Fusion mit einem viralen Gen vorliegen. In diesem Fall wird das immunogene Epitop in Verbindung mit dem viralen Genprodukt exprimiert und es kann eine Immunantwort gegen das zusätzliche immunogene Epitop induziert werden.

Der Vorteil bei der Verwendung von BACs ist, daß eine Gesamtvirus-DNA in die BACs kloniert werden kann oder zumindest größere Teile der viralen DNA. Enthält die Virus-DNA zusätzlich weitere DNA-Abschnitte, die für ein oder mehrere zusätzliche Genprodukte kodieren, so führt die Virusvermehrung auch zu einer Vermehrung des entsprechenden zusätzlichen DNA-Abschnitts, der für ein oder mehrere zusätzliche Genprodukte kodiert. Handelt es sich bei diesen zusätzlichen Genprodukten um therapeutische Genprodukte, wie z.B. Wachstumsfaktoren, Hormone, Enzyme, Impfantigene, Cytokine, immunmodulatorische Proteine, Antisense-RNA-Moleküle und Ribozyme, so wird zusammen mit der Virusvermehrung auch die genetische Information für diese Genprodukte vermehrt. Sind die Expressionskassetten für diese zusätzlichen Genprodukte funktionell, so führt die Virusvermehrung zu einer starken Expression der entsprechenden zusätzlichen Genprodukte, wie z.B. der Wachstumsfaktoren, Hormone, Enzyme, Impfantigene, Cytokine, immunmodulatorischen Proteine, Antisense-RNA-Moleküle und Ribozyme.

Das erfindungsgemäße Vektorsystem kann als pharmazeutische Zusammensetzung formuliert werden. Eine derartige Zusammensetzung enthält ein erfindungsgsgemäßes Vektorsystem zusammen mit üblichen pharmazeutischen Hilfsstoffen. In einer besonderen Ausführungsform der Erfindung handelt es sich bei der Zusammensetzung um eine Zusammensetzung in flüssiger Form. Hierbei kann es sich um flüssiges Bakteriennährmedium handeln, das das erfindungsgemäße Vektorsystem enthält. Dem Fachmann sind geeignete Bakteriennährmedien bekannt. Die flüssige Zusammensetzung kann bei Raumtemperatur aufbewahrt werden. Alternativ kann die flüssige Zusammensetzung auch Zusatzstoffe (z.B. Glycerin) enthalten, die es ermöglichen, die flüssige Zusammensetzung bei Temperaturen unter 0°C aufzubewahren. In einer weiteren bevorzugten Ausführungsform liegt die Zusammensetzung in trockener Form vor.

Verfahren zur Herstellung getrockneter Bakterien sind dem Fachmann bekannt (Billi, D., Wright, D.J., Helm, R.F., Prickett, T., Potts, M., and Crowe, J.H., Appl. Environ. Microbiol. (2000), 66, 1680-1684; Welsh D.T. and Herbert R.A., FEMS Microbiol. Lett. (1999), 174, 57-63 und Janning B., in t'Veld, P.H., Notermans, S., and Kramer, J., J. Appl. Bacteriol. (1994), 77, 3219-324).

Wie oben ausgeführt, eignet sich das erfindungsgemäße Vektorsystem in Abhängigkeit der in den BACs enthaltenen heterologen DNA zur Verwendung als Impfstoff oder Arzneimittel. Insbesondere dann, wenn das heterologe Gen ein therapeutisches Gen ist, also beispielsweise ein Enzym, ein Hormon, ein Wachstumsfaktor, ein Cytokin, ein immunmodulatorisches Protein, ein Antisense-RNA-Molekül oder ein Ribozym, eignet sich das Vektorsystem zur Verwendung als Arzneimittel. Exprimiert die heterologe DNA ein Epitop, so kann das Vektorsystem, wie oben ausgeführt, als Impfstoff verwendet werden.

Die erfindungsgemäße Zusammensetzung kann zur therapeutischen Behandlung oder zur Infektion den zu behandelnden Menschen oder Tieren in beliebiger Art und Weise verabreicht werden. Insbesondere bietet es sich allerdings an, das erfindungsgemäße Vektorsystem auf intravenösem, parenteralem, intraperitonealem, subkutanem oder intramuskulärem Wege zu verabreichen. Am meisten bevorzugt wird die erfindungsgemäße Zusammensetzung in Form einer enteralen (z.B. oralen oder rektalen) Zusammensetzung verabreicht. Kodiert die heterologe DNA im künstlichen bakteriellen Chromosom für ein Epitop und eignet sich die erfindungsgemäße Zusammensetzung als Impfstoff, kann somit ein Impfstoff in Form eines Schluckimpfstoffs verabreicht werden. Handelt es sich bei der erfindungsgemäßen Zusammensetzung um eine Zusammensetzung in flüssiger Form, so kann diese direkt verabreicht werden. Handelt es sich bei der erfindungsgemäßen Zusammensetzung um eine Zusammensetzung in trockener Form, so kann diese vor der Verabreichung als Schluckimpfstoff in ein geeignetes Flüssigmedium gegeben werden, so daß das Vektorsystem, d.h. die invasiven Bakterien, die ein geeignetes bakterielles künstliches Chromosom enthalten, in geeigneter Weise reaktiviert wird, bevor es verabreicht wird.

Unabhängig davon, ob eine Verabreichung in trockener oder in flüssiger Form erfolgt, sind bei einer bevorzugten Ausführungsform die invasiven Bakterien, die ein eine heterologe DNA enthaltendes bakterielles künstliches Chromosom umfassen, lebensfähig. Wird das erfindungsgemäße Vektorsystem bzw. die erfindungsgemäße Zusammensetzung zum Zwecke der Impfung verabreicht, so handelt es sich somit bei der erfindungsgemäßen Zusammensetzung um einen Lebendimpfstoff.

Die Vorteile einer derartigen Verabreichung sind insbesondere für die Länder zu sehen, in denen eine Kühlkette, die für herkömmliche Impfstoffe notwendig ist, nicht immer eingehalten werden kann. In diesen Ländern könnte die trockene oder die flüssige Zubereitung in einfacher Weise transportiert und gelagert werden, ohne daß es in jedem Fall und ständig notwendig wäre, eine geeignete Kühleinreichtung bereitzustellen.

Gegenüber den sonstigen bekannten Verfahren zum Einbringen von DNA in eukaryonte Organismen hat das erfindungsgemäße Vektorsystem eine Reihe von Vorteilen. So sind im Stand der Technik einige Verfahren zum Einbringen von nackter DNA in lebende Organismen beschrieben worden. Dabei wurde die nackte DNA entweder injiziert oder auf parenteralem Weg verabreicht. All diese Methoden setzen jedoch die Aufreinigung großer Mengen an DNA guter Qualität und aufwendige DNA-Transferprotokolle voraus. Oft ist die transferierte DNA-Menge sehr klein, was zu einer geringen Antigenexpression führen kann. Letztere kann dann sogar Toleranz gegenüber dem eingeführten Antigen und nicht eine Immunisierung erzeugen. Werden dagegen die in einer der bevorzugten Ausführungsformen beschriebenen, selbst replizierenden viralen Vektoren verwendet, so reichen auch kleinere transferierte DNA-Mengen aus. Allerdings mußten im Stand der Technik zu diesem Zweck auch aufwendige DNA-Präparationen vorgenommen werden. Bei der vorliegenden Erfindung sind derartige aufwendige Transferverfahren für DNA und insbesondere aufwendige DNA-Aufbereitungsverfahren nicht mehr notwendig, da die einzuführende DNA mittels invasiver Bakterien verabreicht werden kann.

Die Erfindung betrifft auch Verfahren zur Herstellung von Medikamenten zur Behandlung von Krankheiten, bei denen die Expression von eingeführten Genen eine therapeutische Wirkung haben kann. Hierunter fallen insbesondere Tumor- und Krebserkrankungen, bei denen beispielsweise Suizidgene zum Abtöten der Krebszellen führen können. Weiterhin fallen darunter beispielsweise genetische Erkrankungen, bei denen durch das Fehlen einer bestimmten Genfunktion Krankheitssymptome entstehen. Beispiele hierfür sind z.B. Defekte in der Adenosindesaminase (SCID), in Blutgerinnungsfaktoren (Haemophilie), dem LDL-Rezeptor (familiäre Hypercholesertinaemie), oder der UDP-Glucuronosyltransferase (Crigler-Najjar Syndrom).

Das Vektorsystem kann auch geeignet sein, um Erkrankungen zu therapieren, bei denen bestimmte Genfunktionen überexprimiert werden. In diesem Fall sollte das mit dem Vektorsystem eingeführte heterologe Gen eine Antisense-RNA oder ein Ribozym exprimieren, das mit der RNA, die für das überexprimierte Genprodukt kodiert, interagiert.

Die Erfindung betrifft weiterhin die Verwendung des erfindungsgemäßen Vektorsystems zur Herstellung von Medikamenten, die als Impfstoff geeignet sind. In diesem Fall soll in dem Vektorsystem das BAC als heterologe DNA eine DNA enthalten, die für mindestens ein immunogenes Epitop kodiert.

Bei einer besonders bevorzugten Ausführungsfrom sind die Gene, die für therapeutische oder immunogene Genprodukte kodieren, im Virus-DNA-Anteil eines Virus-DNA enthaltenden BACs enthalten.

Die Erfindung betrifft außerdem neben der Verwendung des erfindungsgemäßen Vektorsystems zur Herstellung von Medikamenten, die als Arzneimittel oder Impfstoffe wirken, auch die Verwendung des Vektorsystems für entsprechende therapeutische oder impftechnische Anwendungen selbst.

Die Erfindung betrifft weiterhin eukaryonte Zellen, die ein erfindungsgemäßes Vektorsystem enthalten. In diesem Zusammenhang wird, abweichend vom sonstigen Gebrauch des Begriffs "eukaryonte Zelle" in dieser Anmeldung, unter eukaryonter Zelle nicht der menschliche Gesamtorganismus verstanden.

Invasive Bakterien, die ein bakterielles künstliches Chromosom enthalten, wobei das bakterielle künstliche Chromosom Virus-DNA enthält, können zur Charakterisierung von Virusmutanten verwendet werden. Ausgangspunkt für ein derartiges Verfahren zur Charakterisierung von Virusmutanten ist ein BAC, das ein vollständiges Virusgenom enthält. Insbesondere eignet sich das Verfahren für die Analyse von Genfunktionen in der DNA großer DNA-Viren. Im Rahmen der vorliegenden Erfindung werden unter großen DNA-Viren, insbesondere Herpesviren, am meisten bevorzugt Cytomegaloviren verstanden. Die Virus-DNA kann mittels dem Fachmann bekannter Verfahren vor der Einklonierung in die BACs oder nach der Einklonierung in die BACs mutagenisiert werden. Bei der Mutagenisierung kann es sich einmal um gezielte Mutagenese handeln (siehe z.B. Messerle et al., Proc. Natl. Acad. Sci. USA (1997), 94, 14759-14763 für die Herstellung mutagenisierter Herpesviren). Die Mutagenese kann beispielsweise durchgeführt werden durch homologe Rekombination zwischen der Virus-DNA im BAC und einem Plasmid, das ebenfalls Virus-DNA enthält, die homolog zu dem Bereich ist, in den ein weiterer DNA-Abschnitt eingeführt werden soll. Auf diese Weise kann in gezielter Art und Weise ein bestimmtes Gen durch homologe Rekombination und Insertion eines weiteren DNA-Abschnitts zerstört werden. Ein Verfahren zur Mutation von Virus-DNA unter Verwendung von PCR-Fragmenten wird weiter unten beschrieben. Alternativ kann auch eine randomisierte Mutagenese stattfinden. Ein Beispiel für ein Verfahren zur randomisierten Mutagenese ist die sogenannte Transposonmutagenese (Brune et al., Nature Biotechnology (1999), 17, 360-364).

Bei einer Ausführungsform der Erfindung wird die Mutagenese in randomisierter Art und Weise durchgeführt und die randomisierten mutagenisierten viralen Genome in einem bakteriellen künstlichen Chromosom befinden sich als "Bank" in Bakterien. Bei Bakterien handelt es sich vorzugsweise um E. coli Bakterien. Unter dem Begriff "Bank" wird verstanden, daß eine Vielzahl unterschiedlich randomisiert mutagenisierter viraler Genome in bakteriellen künstlichen Chromosomen in Bakterien vorliegt. In diesem Sinn kann eine "Bank" auch definiert werden als eine heterogene Sammlung an mutierten, Virus-DNA enthaltenden BACs. In diese Bank können nun ein oder mehrere Gen(e) eingebracht werden, die dazu führen, daß ein natürlicherweise nicht-invasives Bakterium zu einem invasiven Bakterium wird. Wie bereits oben ausgeführt, kann es sich hierbei um das inv-Gen von Yersinia pseudotuberculosis oder Yersinia enterocolitica und gegebenenfalls das hly-Gen von Lysteria monocytogenes handeln. Diese Gene liegen vorzugsweise auf einem oder mehreren Plasmiden. Alternativ kann es sich bei den Bakterien, die die Bank randomisiert mutagenisierter viraler Genome in einem bakteriellen künstlichen Chromosom enthalten, auch um Bakterien handeln, die natürlicherweise invasiv sind.

Grundsätzlich können alle Bakterien verwendet werden, die entweder natürlicherweise invasiv sind oder die durch das Einführen geeigneter Gene zu invasiven Bakterien werden. Die Mutagenese kann entweder in den invasiven Bakterien vorgenommen werden oder aber die Mutagenese wird in nicht-invasiven Bakterien vorgenommen und die so mutagenisierten BACs können dann in die invasiven Bakterien eingebracht werden. Alternativ dazu kann die Mutagenese in nicht-invasiven Bakterien vorgenommen werden und die nicht-invasiven Bakterien können dann, wie oben ausgeführt, durch die Einführung geeigneter Gene in invasive Bakterien umgewandelt werden. Maßgeblich ist lediglich, daß nach Beendigung dieser ersten Stufe eine Bank randomisierter mutagenisierter viraler Genome in einem bakteriellen künstlichen Chromosom in invasiven Bakterien vorliegt.

In der zweiten Stufe des Verfahrens können die erhaltenen Bakterien dann vermehrt werden. In der dritten Stufe kann auf Bakterien selektiert werden, die sowohl invasive Bakterien sind als auch randomisiert mutagenisierte virale Genome in einem BAC enthalten. Handelt es sich bei den Bakterien natürlicherweise um invasive Baktieren, ist in bezug auf das Vorhandensein der entsprechenden Gene kein zusätzlicher Selektionsschritt notwendig. Wurden natürlicherweise nicht-invasive Bakterien mit einem oder mehreren Plasmiden transfiziert, die Gene enthalten, die natürlicherweise nicht-invasive Bakterien in invase Bakterien umwandeln, so ist es notwendig, daß die entsprechenden Plasmide einen geeigneten Selektionsmarker enthalten. Das Vorhandensein von BACs kann dadurch sichergestellt werden, daß das verwendete BAC einen Selektionsmarker enthält, der verschieden sein sollte von dem Selektionsmarker, der dazu verwendet wird, um auf das Vorhandensein von Plasmiden zu selektionieren, die die für die Herstellung von invasiven Bakterien notwendigen Genprodukte exprimieren. Wird die Mutagenese so durchgeführt, daß zusätzliche Gene in die Virus-DNA eingeführt werden (beispielsweise durch Transposonmutagenese), so kann mit diesen zusätzlichen Genen ein dritter Selektionsmarker eingeführt werden, so daß unabhängig voneinander auf das Vorhandensein von mutagenisierter Virus-DNA, BAC und Plasmiden, die Invasionsgenprodukte enthalten, selektioniert werden kann.

Durch die Mutagenese der Virus-DNA in dem BAC kann unter Umständen eine virale Genfunktion zerstört worden sein, die für die Replikation und/oder Vermehrung des Virus notwendig ist. In einem weiteren Schritt wird es i.d.R. deshalb zunächst notwendig sein, auf lebensfähige Viren zu selektieren. Eine Selektion auf lebensfähige Viren erfolgt beispielsweise dadurch, daß die invasiven Bakterien enthaltend die BACs mit den mutierten Virusgenomen auf eukaryonte Zellen gegeben werden, die für das entsprechende Virus permissiv sind, in denen es also zu einer Virusvermehrung kommt.

Handelt es sich bei der mutierten Virus-DNA um Cytomegalovirus-DNA, können die Bakterienklone beispielsweise zu Fibroblasten gegeben werden. Eine Virusvermehrung kann beispielweise durch Ausbildung eines cytopathischen Effekts oder Lyse der Zellkultur festgestellt werden. Wurde die Mutagenese so durchgeführt, daß ein Markergen, wie z.B. das Gen für das grün fluoreszierende Protein in die Virus-DNA eingeführt wurde, so kann die Virusvermehrung beispielsweise auch über das Vorhandensein des Markergens überprüft werden, also z.B. durch den Nachweis des grün fluoreszierenden Proteins.

Die lebensfähigen Virusmutanten können dann in einem geeigneten Testsystem analysiert werden. Beispielsweise kann überprüft werden, welche Viren eine unterschiedliche Vermehrung auf bestimmten Zellen zeigen oder welche Viren gegenüber bestimmten Medikamenten besonders wirksam sind. Mittels dem Fachmann bekannter Methoden kann dann analysiert werden, an welcher Stelle eine Mutagenese erfolgte, falls es sich um eine randomisierte Mutagenese handelte (Brune et al. Nature Biotechnology (1999), 17, 360-364). Auf diese Art und Weise können Rückschlüsse darauf gezogen werden, welche Genfunktion für welchen Effekt verantwortlich ist.

Gemäß der im Stand der Technik bekannten Verfahren muß für eine Rekonstitution von Virus nach der Transposonmutagenese eine ausreichende Menge an BAC-DNA, die die Virus-DNA enthält, aus Bakterien präpariert und in eukaryontische Zellen überführt werden. Um die Phänotypen einer großen Anzahl an Virus-mutanten, die mit einer ungezielten Mutagenesemethode hergestellt wurden, zu bestimmen, ist also gemäß der im Stand der Technik bekannten Verfahren die aufwendige DNA-Präparation und Virusrekonstitution durch Transfektion unausweichlich notwendig. Eine kostengünstigere und schnellere Möglichkeit der Rekonstitution vieler Virusmutanten ist dagegen mit dem oben beschriebenen, erfindungsgemäßen Verfahren möglich, da in diesem Fall die Bakterien, die neben dem mutagenisierten, Virus-DNA enthaltenden BAC auch spezifische Gene für die Invasion von eukaryontischen Zellen besitzen, einfach auf die Zellen in den Zellkuturen gegeben werden und nach einigen Tagen bzw. Wochen entsteht Virus ohne DNA-Präparation oder Transfektion der Zellen.

Erfindungsgemäß wird weiterhin ein Verfahren zum ortsspezifischen Einfügen eines heterologen DNA-Abschnitts in Virus-DNA enthaltende BACs bereitgestellt. Enthalten bakterielle künstliche Chromosomen das Genom großer DNA-Viren (z.B. das 36 kbp-Genom von Adenoviren oder das 230 kbp-Genom von Cytomegaloviren), so kann in die Virus-DNA enthaltenden BACs in Baktieren nicht durch gängige Methoden, wie z.B. durch Restriktionsspaltungen und Ligation von DNA-Fragmenten, kloniert werden. Daher ist das Einführen von Mutationen oder das Einbringen von Fremdgenen in die Virus-DNA enthaltenden BACs durch homologe Rekombination die derzeit einzige effiziente Mutagenesemethode. Dabei findet die homologe Rekombination statt zwischen der Virus-DNA im BAC und einem DNA-Fragment, das den zu inserierenden DNA-Abschnitt umfaßt und das von viralen Sequenzen flankiert ist, die homolog zum gewünschten Insertionsort in der Virus-DNA sind. Hier sind im Stand der Technik drei Verfahren bekannt:

Bei dem ersten Verfahren handelt es sich um die Transposonmutagenese für die ungezielte Unterbrechung von Genen. Diese Mutation ist insbesondere beschrieben in Brune et al., Nature Biotechnology (1999), 17, 360-364. Dieses Verfahren dient der Charakterisierung von viralen Genfunktion, ist aber zur zielgerichteten Mutagenese (z.B. Einführen von Epitopsequenzen zur Immunisierung) nicht geeignet.

Bei dem zweiten Verfahren handelt es sich um eine Zwei-Stufen-Rekombinationsmethode über Shuttle-Plasmide. Diese Methode ist ausführlich beschrieben in der Patentanmeldung PCT/EP98/04816 und in der Publikation von Messerle et al., Proc. Natl. Acad. Sci. (1997),94(26), S. 14759-14763.

Bei diesem Verfahren kann jede Art von Mutation (Deletion, Insertion von Fremdgenen, Punktmutation) eingefügt werden. Das Verfahren erfordert aber die Klonierung eines Shuttle-Plasmids, das die mutierte DNA-Sequenz und flankierende homologe Sequenzen von mindestens 2 kbp zu dem mutierten Gen trägt.

Da die notwendige Subklonierung der viralen DNA-Homologien das Vorhandensein von geeigneten Restriktionsschnittstellen voraussetzt, ist ein nukleotidgenaues Einfügen der Mutation mit dieser Methode nicht möglich. Zudem erfordert eine in-frame-Fusion eines Fremdgens in einem viralen Leserahmen zusätzlich eine vorherige PCR-Mutagenese des subklonierten Gens auf Plasmidebene, was die Mutageneseprozedur sehr kompliziert und zeitaufwendig macht.

Bei dem dritten Verfahren handelt es sich um die Doppelrekombination mit einem linearen DNA-Fragment durch ET-Rekombinasen. Hierbei erfolgt eine Doppelrekombination zwischen der zu mutierenden DNA mit einem linearen Fragment, das die mutierte DNA-Sequenz und flankierende homologe Sequenzen von ca. ***30*** bis 60 bp umfaßt. Die Rekombination wird durch das ET-Rekombinasesystem vermittelt (WO 99/29837, Zhang et al., Nature Genetics (1998), 20(2), 123-128).

Obwohl diese Methode die Herstellung einer Deletionsmutante in wenigen Tagen und nukleotidgenau ermöglicht, erfordert das Inserieren von Fremd-DNA (Epitopen, Genen, usw.) oder das Einführen von Punktmutation mit dieser Methode eine zweite Mutageneserunde. Die notwendige Gegenselektion gegen einen Negativmarker (z.B. SacB) ist zudem sehr ineffizient und für manche virale Genome (wie z.B. dem murinen CMV (mCMV)) aufgrund von Instabilität des Genoms nicht anwendbar.

Die vorliegende Erfindung ermöglicht nun unter anderem die nukleotidgenaue (in-frame) Insertion von DNA-Sequenzen mit einer einzigen Transformation, ohne die Notwendigkeit einer Klonierung in das Virus-DNA enthaltende BAC. So umfaßt die Erfindung ein Verfahren zum ortsspezifischen Einfügen eines heterologen DNA-Abschnitts in Virus-DNA enthaltende BACs, umfassend die folgenden Stufen:
(i) Zunächst wird eine PCR durchgeführt. Dazu wird ein Senseprimer A und ein Antisenseprimer B verwendet. Bei einem "Primer" handelt es sich um ein synthetisch hergestelltes Oligodesoxyribonukleotid. Verfahren zur Herstellung derartiger Primer sind dem Fachman bekannt. Die erfindungsgemäß verwendeten Primer A und B können eine Länge von bis zu ca. 120 Basenpaare haben. Solche Primer können kommerziell bei Firmen, die Oligodesoxyribonukleotid-Synthesen betreiben, erworben werden. Die 5'-Enden beider Primer sind homolog zu der Zielregion der Virus-DNA, in die der heterologe DNA-Abschnitt eingefügt werden soll. Die 3'-Enden der Primer A und B sind dagegen homolog zu den Enden des einzuführenden DNA-Abschnittes. Als Matrize für die PCR wird eine DNA verwendet, die den einzuführenden DNA-Abschnitt enthält. Wird die PCR auf diese Art und Weise durchgeführt, erhält man somit ein doppelsträngiges DNA-Fragment, das an beiden Enden Regionen hat, die homolog sind zu den Bereichen in der Virus-DNA, in die durch homologe Rekombination der heterologe DNA-Abschnitt eingeführt werden soll. Zwischen diesen Bereichen, die homolog zu der Zielregion sind, befindet sich dagegen der heterologe DNA-Abschnitt. Bei diesem heterologen DNA-Abschnitt kann es sich um beliebige DNA handeln, beispielsweise einen Selektionsmarker, wie ein wie oben definiertes Resistenzgen, ein Gen das für das grün fluoreszierende Protein, die β-Galactosidase etc. kodiert. Am meisten bevorzugt handelt es sich bei dem Selektionsmarker um ein Resistenzgen. Das Gen, das für einen Selektionsmarker kodiert, kann dabei von Sequenzen flankiert sein, die Erkennungssequenzen für sequenzspezifische Rekombinasen darstellen oder aber Erkennungssequenzen für Restriktionsendonukleasen sind, die sich von den Sequenzen des restlichen Vektors unterscheiden. Insbesondere kann es sich bei den Erkennungsstellen für sequenzspezifische Rekombinasen um loxP-Sequenzen oder FRT-Sequenzen handeln. Diese dienen als Erkennungssequenzen für die Rekombinasen Cre bzw. FLP. Die Rekombinasen vermitteln über Rekombination das Herausschneiden der DNA-Sequenz, die zwischen gleich orientierten loxP- bzw. FRT-Sequenzen liegt. Dies ist dem Fachmann bekannt.
   Der mittels PCR eingeführte DNA-Abschnitt kann neben dem Selektionsmarker gegebenenfalls ein weiteres DNA-Fragment enthalten. Bei diesem weiteren DNA-Fragment kann es sich um ein Gen handeln, das für ein wie oben definiertes beliebiges therapeutisches oder immunogenes Genprodukt kodiert. Sollen mit dem erfindungsgemäßen Verfahren mehrere Gene eingeführt werden, z.B. ein therapeutisches oder immunogenes Gen und ein Selektionsmarker, so müssen diese zwei oder mehr Gene auf dem gleichen DNA-Fragment enthalten sein, das als Matrize für die PCR verwendet wird.
   Bei einer bevorzugten Ausführungsform des Verfahrens enthält einer der Primer A oder B, die zur PCR verwendet werden, zwischen dem Bereich, der homolog zur Zielregion der Virus-DNA ist, und dem Bereich, der homolog zur einzuführenden DNA ist, eine zusätzliche Fremd-DNA-Sequenz. Bei dieser zusätzlichen Fremd-DNA-Sequenz handelt es sich bei einer bevorzugten Ausführungsform um eine Sequenz, die für ein immunogenes Epitop oder ein anderes Peptid kodiert. Vorzugsweise ist die Primersequenz so gewählt, daß im nach der Mutagenese erhaltenen rekombinanten BAC die DNA-Sequenz für das immunogene Epitop oder das kurze Peptid im gleichen Leseraster liegt wie ein virales Gen. Somit wird das virale Genprodukt das zusätzliche immunogene Epitop oder das kurze Peptid enthalten. Bei dem kurzen Peptid kann es sich beispielsweise um ein Flag-tag oder HA-Tag handeln. Eine erfolgte Rekombination kann unter anderem durch Sequenzierung der Epitop-Insertionsstellen mit geeigneten Oligonukleotiden nachgewiesen werden. Zudem kann eine gegebenenfalls erfolgte Rekombinantion (siehe unten) unter anderem dadurch nachgewiesen werden, daß das Vorhandensein des immunogenen Epitops oder des kurzen Peptids in dem entsprechenden viralen Genprodukt nachgewiesen wird. Verfahren zum Nachweis immunogener Epitope und sind dem Fachmann bekannt. Beispielsweise kann das Vorhandensein von Epitopen dadurch nachgewiesen werden, daß die gebildeten Proteine im Western blot oder in Immunpräzipitationsversuchen mit einem polyclonalen oder monoclonalen Antikörper gegen das entsprechende Epitop inkubiert werden. Mittels geeigneter, dem Fachmann bekannter Farbreaktionen kann das Vorhandensein derartiger Komplexe zwischen Antikörpern und Epitop nachgewiesen werden.
   Verfahren zum Einbringen von PCR-Produkten in ein Bakterium sind dem Fachmann bekannt. Es können beispielsweise übliche Transfektionsmethoden, wie z.B. die Calcium-Chlorid-Methode oder Elektroporation, verwendet werden.
   Das erfindungsgemäße Verfahren kann für jede Virus-DNA, die in einem BAC enthalten ist, durchgeführt werden.
   Bei einer bevorzugten Ausführungsform des Verfahrens handelt es sich bei der Virus-DNA allerdings um die DNA eines Herpesvirus, am meisten bevorzugt um die DNA eines Cytomegalovirus.
(ii) Das so hergestellte PCR-Produkt wird in ein Bakterium eingebracht, das ein bakterielles artifizielles Chromosom enthält, welches die Virus-DNA enthält, in die in artspezifischer Weise ein heterologer DNA-Abschnitt eingeführt werden soll. Die Virus-DNA muß einen Abschnitt enthalten, der homolog ist zu den 5'-Enden der Primer A und B.
   Bei dem Bakterium handelt es sich vorzugsweise um E. coli. Bei einer bevorzugten Ausführungsform ist der E. coli-Stamm RecA negativ. Bei einer bevorzugten Ausführungsform exprimiert der verwendete E. coli-Stamm die Gene redα, redβ und redγ des Rekombinationsystems des Phagen γ (Murphy, K.C., J. Bacteriol. (1998), 180, 2063-2071) oder die bakteriellen recE- und recT-Proteine und gegebenenfalls das redγ-Protein (Zhang et al., Nat. Genet. (1998), 20, 123-128). Bei einer bevorzugten Ausführungsform können die recE- und recT-Gene und gegebenenfalls das redγ-Gen auf einem Plasmid liegen. Bei einer weiteren bevorzugten Ausführungsform können die redα-, redβ- und redγ-Gene auf einem Plasmid liegen.
   Bei einer weiteren bevorzugten Ausführungsform der Erfindung handelt es sich bei dem Bakterium um ein invasives Bakterium. Bei einer weiteren Ausführungsform der Erfindung handelt es sich bei dem Bakterium um ein nicht-invasives Bakterium, das jedoch auf die oben beschriebene Weise in ein invasives Bakterium umgewandelt wird (Transfektion des Bakteriums mit Plasmiden, die das inv- und das hly-Gen exprimieren).
(iii) In der letzten Stufe des Verfahrens kann auf Rekombinanten zwischen den PCR-Fragment und den Virus-DNA enthaltenden BACs selektioniert werden. Wie bereits ausgeführt, kann eine derartige Selektion dann relativ einfach durchgeführt werden, wenn das zur Rekombination hergestellte PCR-Fragment einen Selektionsmarker, insbesondere ein Resistenzgen enhält. Alternativ kann, wie ebenfalls bereits ausgeführt, auf Rekombinanten dadurch selektioniert werden, daß überprüft wird, ob ein gegebenenfalls mit dem Primer eingeführtes Epitop oder ein kurzes Peptid in Fusion mit einem Virusprotein vorliegt. Das Einführen einer Epitopsequenz oder der Sequenz eines kurzen Peptids kann beispielsweise dadurch erzielt werden, daß durch die homologe Rekombination ein Stopcodon eines Gens eliminiert wird und im gleichen Leseraster die Fremd-DNA inseriert wird. Dies kann z.B. verwendet werden, um eine Epitopsequenz an ein virales Gen am Carboxyende zu fusionieren. Zum Nachweis des Epitops können monoclonale oder polyclonale Antikörper verwendet werden, die für das entsprechende kurze Peptid oder Epitop spezifisch sind. Beispiele für derartige kurze Peptide oder Epitope sind z.B. das GP33-41-Epitop (Aminosäuresequenz: KAVYNFAT) aus LCMV oder das Flu NP 366-374-Epitop (Aminosäuresequenz: ASNENMDAM) aus dem Influenza A Virus.

Bei einer bevorzugten Ausführungsform des Verfahrens sind die Primer A und B jeweils in einem Bereich von mindestens 25 bp zu der Zielsequenz in der viralen DNA homolog. Bevorzugt ist eine Homologie von 25 bis 75 bp, bevorzugter 25 bis 65 bp, am meisten bevorzugt 25 bis 55 bp. Bevorzugt sind die Rekombinationsproteine redα, redβ (die die Rekombination von nur 35 bp großen DNA-Homologien ermöglichen) und von redγ (das die Exonucleaseaktivität und damit den Abbau von linearer DNA hemmt) im Bakterium exprimiert.

Das mit dem erfindungsgemäßen Verfahren in die Bakterien durch Transformation überführte DNA-Fragment wird durch eine einzige PCR hergestellt. Die hierbei verwendeten synthetischen Primer sind außergewöhnlich lang und erfordern besondere PCR-Bedingungen, die allerdings dem Fachmann bekannt sind. In diesem Zusammenhang wird auf das dem Fachmann bekannte Touch-Down-PCR-Verfahren verwiesen.

Das erfindungsgemäße Verfahren ist nicht auf das ortsspezifische Einführen eines heterologen DNA-Abschnitts in Virus-DNA beschränkt. Vielmehr kann das Verfahren auch zum Einführen heterologer DNA in beliebige "Ziel-DNA" verwendet werden. In diesem Zusammenhang wird unter "Ziel-DNA" beispielsweise die DNA von Eukaryonten, insbesondere von Pilzen, tierischen, menschlichen und pflanzlichen Zellen und Prokaryonten, wie z.B. Bakterien, aber auch von Archäbakterien verstanden.

In diesem Fall muß die Ziel-DNA nicht in einem BAC cloniert sein. Eine Mutation von Eukaryonten oder Prokaryonten kann in diesem Fall dadurch bewerkstelligt werden, daß das wie oben hergestellte PCR-Fragment in die entsprechende prokaryonte oder eukaryonte Zelle eingebracht wird und dann durch zelluläre Rekombinasen eine homologe Rekombination über die homologen DNA-Abschnitte stattfindet und daß dadurch gegebenenfalls zwischen den homologen Bereichen im PCR-Fragment liegende DNA-Abschnitte in die Ziel-DNA eingeführt werden können.

### Beschreibung der Abbildungen

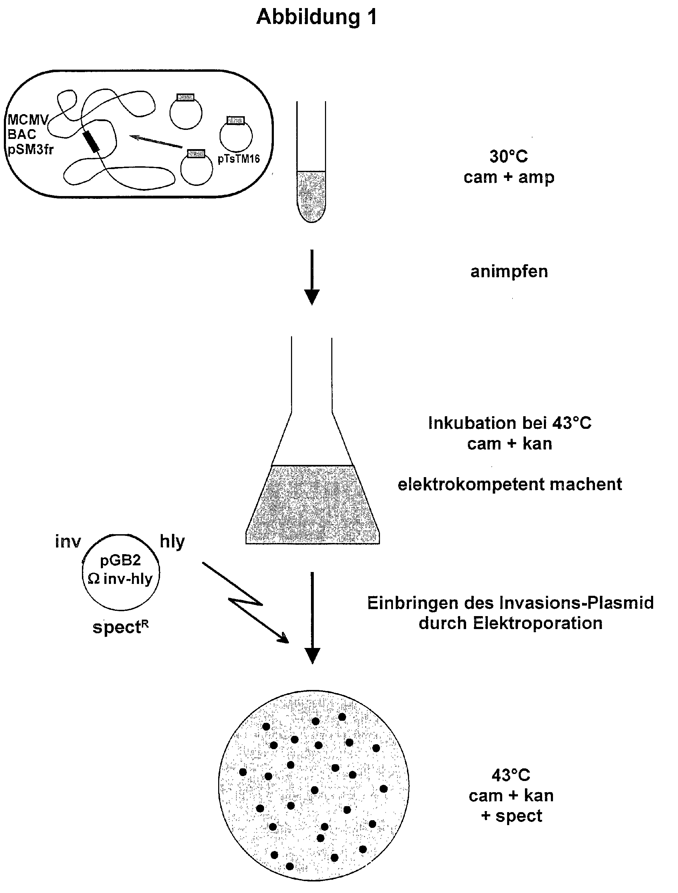

Abb. 1: Herstellung einer Sammlung ("Bank") von MCMV-Transposonmutanten in invasiven E. coli Bakterien
   Dargestellt ist das Einbringen des Transposon-Plasmids pTsTM16 in E. coli Bakterien, die bereits das MCMV-BAC Plasmid pSM3fr (mit dem gesamten MCMV-Genom) besitzen. Durch Selektion auf Chloramphenicol (cam) für das BAC Plasmid und Ampicillin (amp) für das Transposonplasmid und Inkubation bei 30°C werden E. coli Bakterien selektiert, die beide Plasmide besitzen. Dadurch kann es zum "Springen" des Transposons in das MCMV-BAC Plasmid kommen. Diese Bakterienkultur wird daraufhin bei 43°C vermehrt (das Plasmid pTsTM16 wird dadurch entfernt, da es sich nur bei 30°C vermehrt) und elektrokompetent gemacht. In diese Bakterienkultur wird daraufhin das Invasionsplasmid pGB2Qinv-hly eingeführt und mit Spectinomycin darauf selektiert. Dadurch entsteht eine Bakterienmischkultur, die invasive Eigenschaften besitzt.

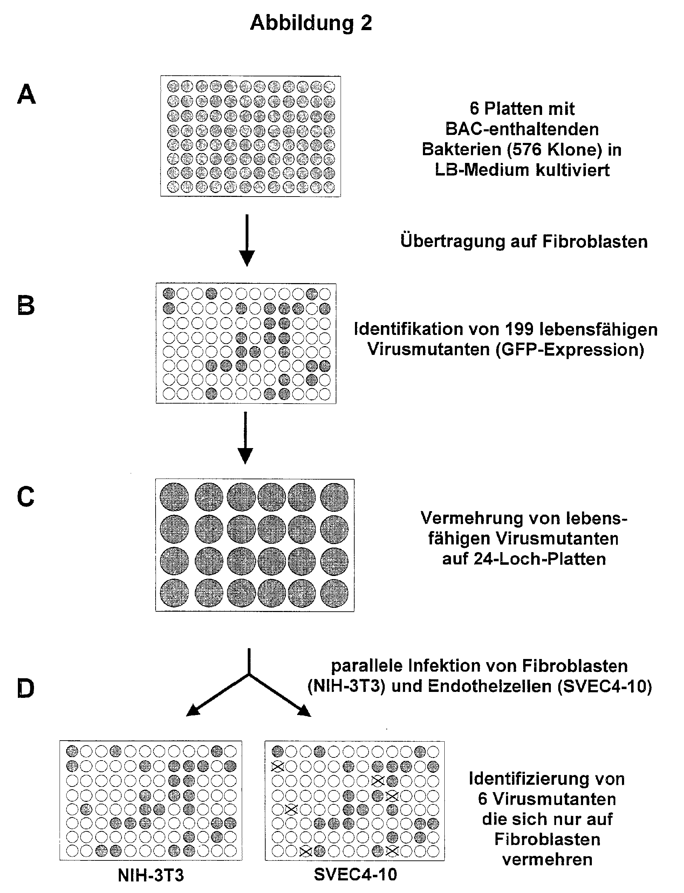

Abb. 2: Suche nach einer MCMV-Mutanten, die nicht auf Endothel-Zellen wächst.
   A. 6 96-Loch-Mikrotiterplatten werden mit einzelnen Bakterienkolonien von E. coli, die ein mutagenisiertes MCMV-BAC Plasmid tragen und invasiv sind, angeimpft. B. 2 *µ*l der Bakterienkultur werden jeweils auf eine Fibroblastenkultur gegeben. Lebensfähige Virusmutanten sind anhand ihrer grünen Fluoreszenz zu erkennen. C. Zur Vermehrung werden die Medium-Überstände mit lebenden Virusmutanten infizierten Zellen zur Vermehrung auf 24-Loch-Mikrotiterplatten mit neuen Fibroblasten gegeben. D. Parallel werden mit der gleichen Virusmutante NIH-3T3- bzw. SVEC4-10-Zellen infiziert und Viruswachstum anhand der grünen Fluoreszenz bestimmt.
Abb. 3: Fluoreszenzmikroskopische Aufnahmen von Zellen
   Endothel-Zellen und Fibroblasten wurden jeweils mit der Virusmutante IE7 und Wildtyp MCMV infiziert und 2 bzw. 6 Tage nach Infektion optisch die Stärke der grünen Fluoreszenz als Maß für Virusvermehrung bestimmt. Die Virusmutante zeigt ein vermindertes Wachstum auf Endothel-Zellen.
Abb. 4: Virusvermehrung auf Endothel-Zellen und Fibroblasten von Tag 0 bis Tag 6 nach Infektion
   Mittels Plaque-Assay wurde die Virusvermehrung zweier ausgewählter Virusmutanten (offenes Viereck und Dreieck) im Vergleich zu Wildtyp MCMV (schwarzes Viereck) verglichen. Auf Fibroblasten (NIH-3T3 fibroblasts) ist nur ein geringer Unterschied in der Virusproduktion zu sehen. Auf Endothel-Zellen (SVEC-4-10 endothelial cells) vermehren sich die Virusmutanten im Vergleich zu Wildtyp MCMV nicht. Jeder schwarze Teilstrich auf der X-Achse entspricht einem Tag.

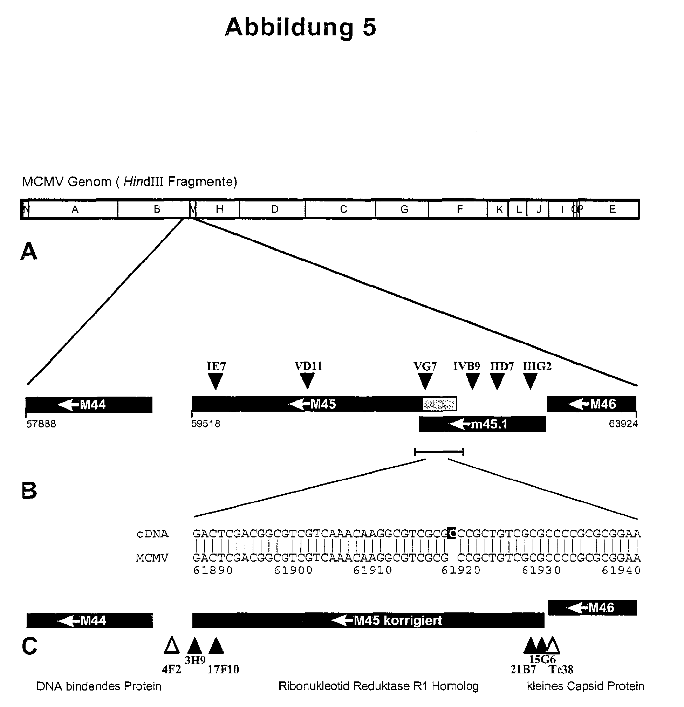

Abb. 5: Schematische Darstellung der Genomorganisation, in der alle 6 Virusmutanten mit vermindertem Wachstum auf Endothel-Zellen eine Transposoninsertion aufweisen
   A. Das MCMV-Genom ist in Abschnitte entsprechend der vorkommenden HindIII-Restriktionsschnittstellen unterteilt. Die Transposoninsertionen (schwarze Pfeile) befinden sich in den Genen M45 und m45.1. C. Sequenzierung des gesamten Genombereichs ergab eine zur veröffentlichten Sequenz abweichende DNA-Sequenz. Nach Korrektur der DNA-Sequenz ergibt sich nur noch ein Leserahmen M45, der Homologien zur Ribonukleotid Reduktase R1 aufweist. Alle Transposoninsertionen sind in oder kurz vor/nach diesem Gen.

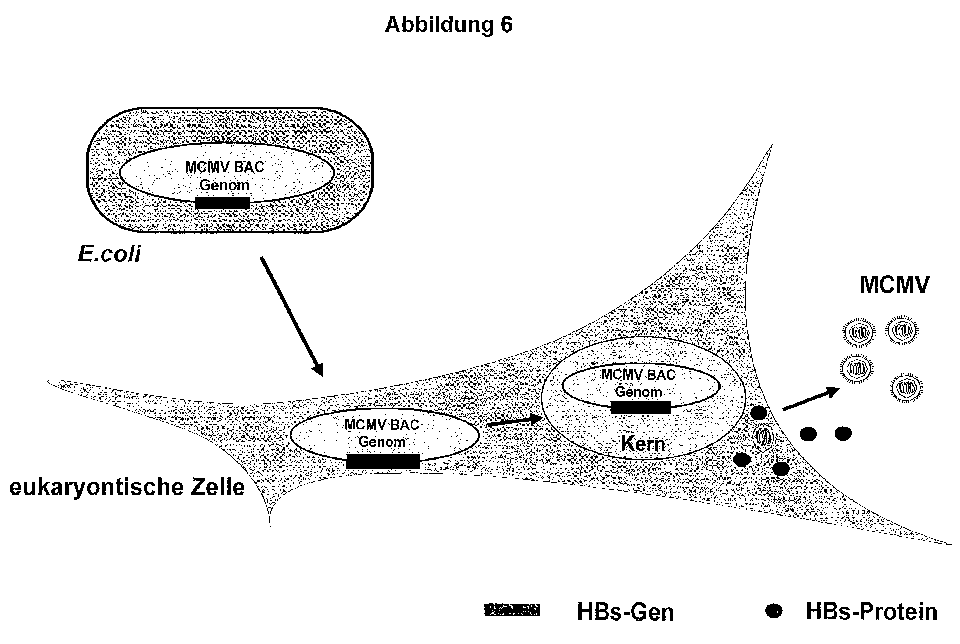

Abb. 6: Schematische Darstellung der Virusrekonstitution aus dem MCMV-BAC Plasmid, das das HBs-Gen zusätzlich inseriert hat
   Durch Zugabe von inasiven E. coli Bakterien (invasive Eigenschaften vermittelt durch vorheriges Einführen des Invasionsplasmids pGB2Ωinv-hly - nicht dargestellt), die das MCMV-HBs BAC Plasmid besitzen, auf eukaryontische Zellen kann durch Aufnahme der Bakterien das MCMV-HBs BAC Plasmid in den Zellkern gelangen. Es kommt zur Virusvermehrung und zur Expression des HBs-Proteins.

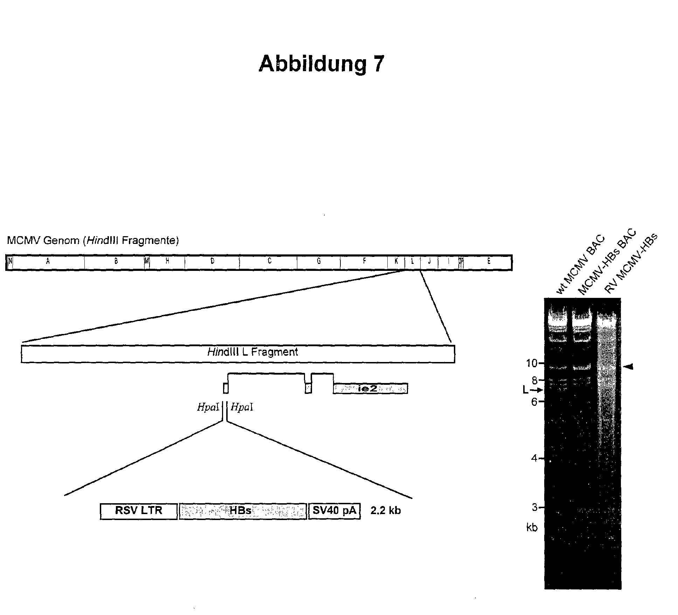

Abb. 7: Schematische Darstellung der Genomregion des MCMV, in der das HBs-Gen inseriert wurde
   Das HBs-Gen mit RSV-LTR-Promotor und Polyadenylierungssignal (SV40 pA) wurde in das Hind L Fragment des MCMV-Genoms integriert. Erfolgreiche Mutagenese zeigte ein neu entstandenes DNA-Fragmente nach HindIII-Restriktionverdau des MCMV-HBs BAC Plasmids und isolierter Virus-DNA (RV MCMV-HBs)bei 9,7 kb und den Verlust einer 7,5 kbp großen DNA-Bande im Vergleich zu DNA des Wildtyp (wt) MCMV-BAC Plasmids (Pfeile).

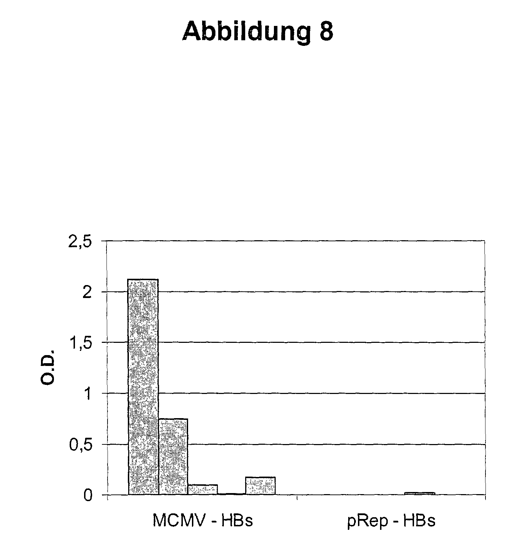

Abb. 8: Konzentration an HBs-Antigen im Serum von intraperitoneal infizierten Mäusen
   HBs-Antigen-Konzentrationen (gemessen anhand der optischen Dichte (O.D.) im ELISA-Test von jeweils 5 intraperitoneal mit MCMV-HBs-BAC- bzw. Kontrollplasmid pREP4-HBs (pRep-HBs) tragenden E. coli Bakterien immunisierten Mäusen. 20 Tage nach Immunisierung. Die Mäuse, die mit MCMV-HBs-BAC-tragenden/invasiven E. coli immunisiert wurden, zeigen signifikante HBs-Antigen-Konzentrationen im Serum. Jede Säule represäntiert eine Maus.

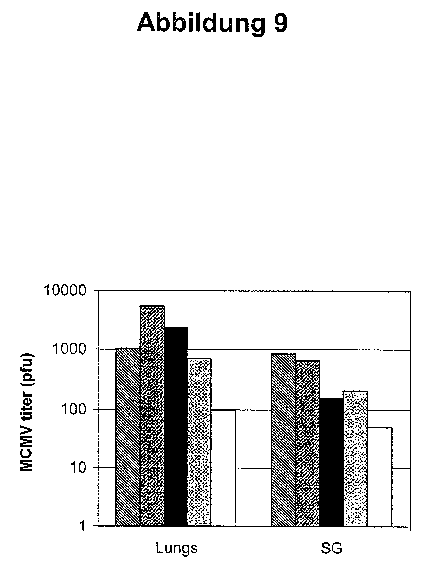

Abb. 9: MCMV-Titer in Lunge und Speicheldrüse von mit MCMV-BAC tragenden E. coli Bakterien infizierten Mäusen
   Die im Ausführungsbeispiel 2 unter Punkt F) beschriebenen infizierten Mäuse wurden am 21. Tag nach Immunisierung getötet und die MCMV-Titer (in PFU= Plaque bildende Einheiten) in Lunge (bezeichnet als Lungs) und Speicheldrüse (SG) mit Standard-Plaque-Assay in MEF bestimmt. Organhomogenisate von Lungen (Lungs) und Speicheldrüsen (SG) der Mäuse, die intraperitoneal mit MCMV-HBs BAC und pGB2Ωinv-hly Plasmide tragenden E. coli infiziert wurden, wurden verwendet. Jede Säule stellt eine Maus dar.

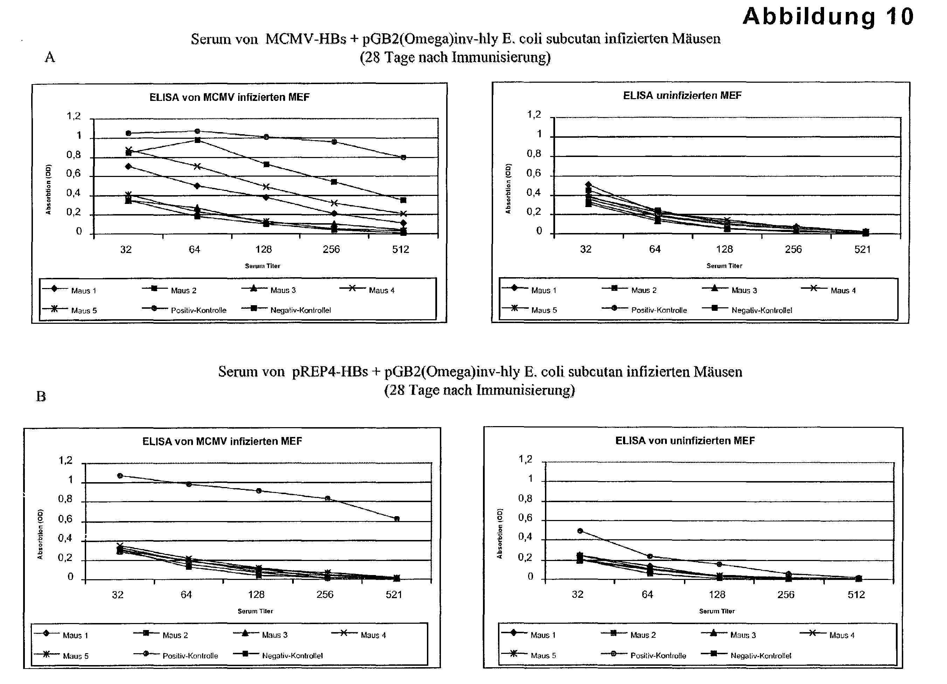

Abb. 10: Virustiter von subkutan mit E. coli (enthaltend pGB2Ωinv-hly und MCMV-HBs-BAC) infizierten Mäusen im Serum
   Den im Ausführungsbeispiel 2 unter Punkt I) beschriebenen subkutan mit E. coli immunisierten Mäusen wurde 28 Tage nach Immunisierung Serum entnommen und der Gehalt an gebildeten Antikörpern gegen MCMV im ELISA-Test gemessen. Die Konzentration an anti-MCMV-Antikörpern ist anhand der Adsorbtion (OD) für ansteigende Serum Titer (32 bis 521) für alle 5 Mäuse und einer internen Negativ- und Positiv-Kontrolle angegeben (für Einzelheiten zum ELISA-Test sieh in Beschreibung angegebene Literatur). Von den Mäusen, die mittels E. coli das MCMV-HBs-BAC Plasmid erhalten haben zeigten 3 signifikante Titer an anti-MCNV-Antikörpern (A). Mäuse die das Kontrollplasmid pGB2Ω-inv-hly erhalten haben, zeigten keine Antikörper im Serum (B). Rechts in A. und B. sind jeweils die Negativ-Kontrollen mit uninfizierten MEF dargestellt.

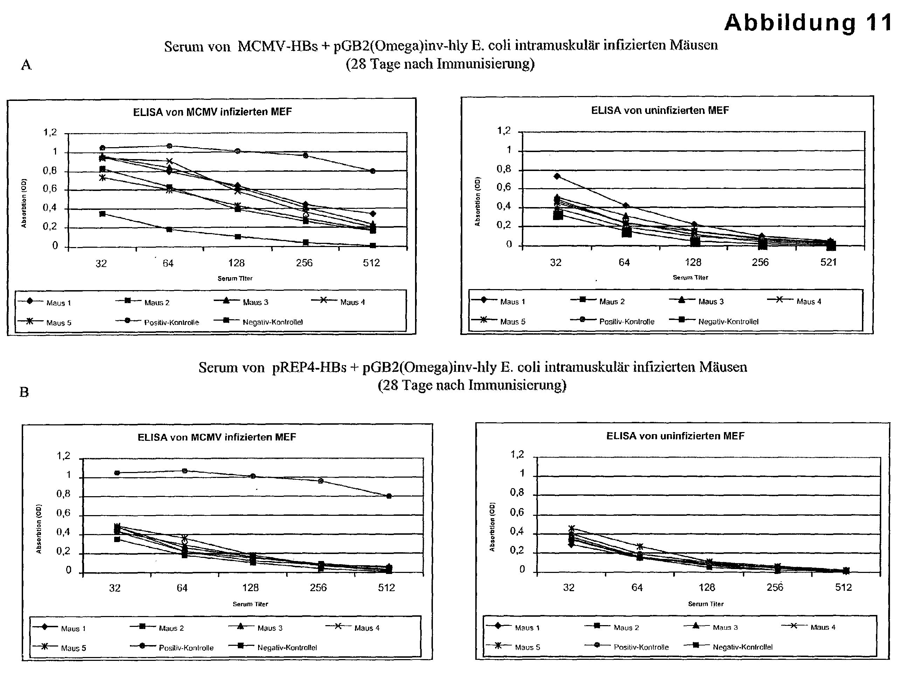

Abb. 11: Virustiter von intramuskulär mit E. coli (enthaltend pGB2Ωinv-hly und MCMV-HBs-BAC) infizierten Mäusen im Serum
   Den im Ausführungsbeispiel 2 unter Punkt I) beschriebenen intramuskulär mit E. coli immunisierten Mäusen wurde 28 Tage nach Immunisierung Serum entnommen und der Gehalt an gebildeten Antikörpern gegen MCMV im ELISA-Test gemessen. Die Konzentration an anti-MCMV-Antikörpern ist anhand der Adsorbtion (OD) für ansteigende Serum Titer (32 bis 521) für alle 5 Mäuse und einer internen Negativ- und Positiv-Kontrolle angegeben (für Einzelheiten zum ELISA-Test siehe die in der Beschreibung angegebene Literatur). Von den 5 Mäusen, die mittels E. coli das MCMV-HBs-BAC Plasmid intramuskulär erhalten haben, zeigten alle signifikante Titer an anti-MCMV-Antikörpern (A). Mäuse die das Kontrollplasmid pGB2Ω-inv-hly erhalten haben, zeigten keine Antikörper im Serum (B). Rechts in A. und B. sind jeweils die Negativ-Kontrollen mit uninfizierten MEF dargestellt.

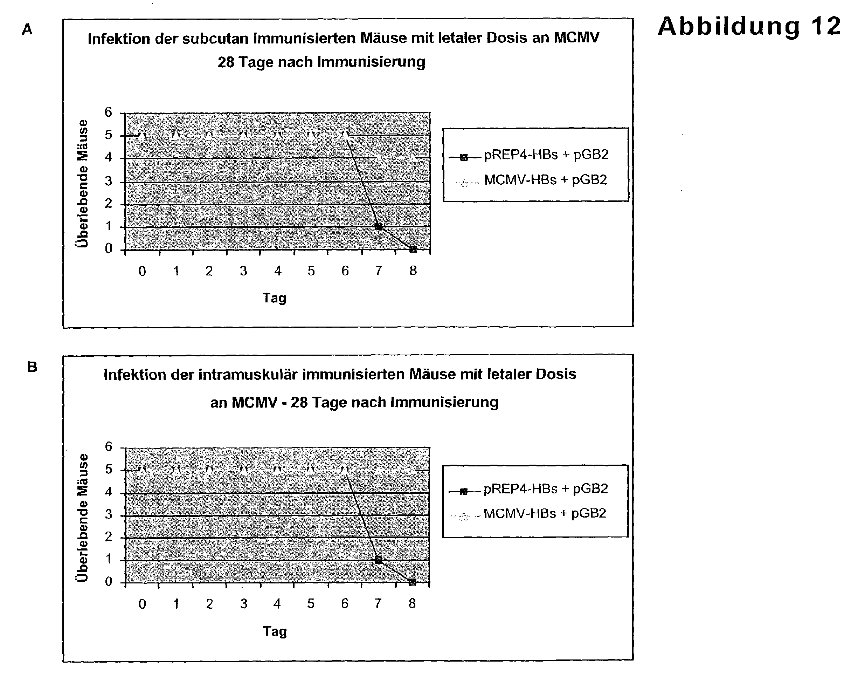

Abb. 12: Die durch MCMV-HBs-BAC tragende invasive E. coli Bakterien immunisierten Mäuse zeigen Immunisierungsschutz vor einer Infektion mit Wildtyp-MCMV
   28 Tage nach der Immunisierung mit invasiven E. coli Bakterien, die das MCMV-HBs-BAC Plasmid trugen, (A = subkutan verabreicht; B = intramuskulär verabreicht) wurden jeweils 5 Mäuse mit einer letalen Dosis an MCMV infiziert. A. Von den subkutan immunisierten Mäusen überlebten 4 von 5 bis zum 8.Tag nach Infektion mit MCMV. Von den mit dem Kontrollplasmid pREP4-HBs immunisierten Mäusen waren 8 Tage nach MCMV-Infektion alle tot. B. Von den auf intramuskulären Weg mittels invasiver E. coli immunisierten Mäusen überlebten alle die MCMV-Infektion, im Gegensatz zu den mit dem Kontrollplasmid immunisierten Mäusen, die alle nach dem 8.Tag tot waren.

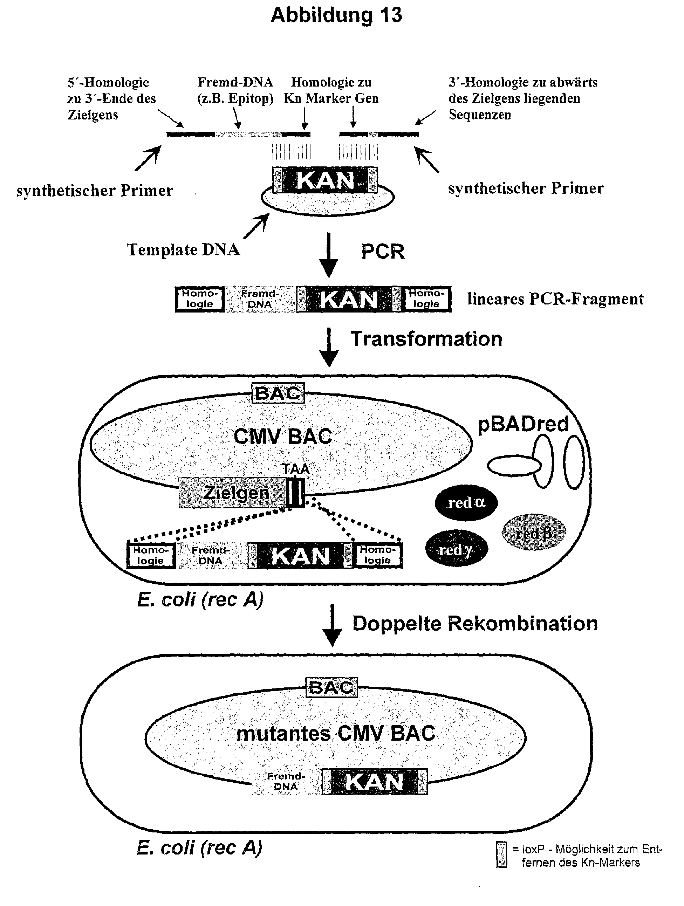

Abb. 13: Prinzip eines 1-Schritt-Verfahrens zur Fusion eines Fremd-Epitops an ein virales Gen im MCMV-BAC Plasmid durch Rekombination mit einem linearen PCR-Fragment, das das Epitop im Primer trägt
   Synthetische Oligonukleotide, die als Primer in einer PCR dienen, können bis zu einer Länge von max. ca. 120 Basenpaaren synthetisch von Firmen hergestellt werden. Die Sequenz ist so zu wählen, daß die Primer eine Homologie aufwärts (5'-Homologie) bzw. abwärts (3'-Homologie) zur gewünschten Insertionsstelle im MCMV-BAC Plasmid tragen. Zusätzlich müßen sie identisch mit dem Anfang und Ende des Kanamycin-Gens sein, um ein "Priming" bei der PCR zu ermöglichen. Das Kanamycin-Gen kann von Loxp-Sequenzen bzw. FRT-Sequenzen flankiert sein, um ein späteres Herausschneiden des Kanamycin-Gens zu ermöglichen. Zur Insertion einer zusätzlichen Epitop-Sequenz, muß einer der Primer zwischen der Homologie zum MCMV-BAC und zur Kanamycin-Gen-Priming-Sequenz noch die DNA-Sequenz des Epitops tragen. Die Template-DNA ist meist das PACYC177-Plasmid (New England Biolabs). Das lineare PCR-Fragment wird entsprechend der Text-Beschreibung behandelt und in E. coli überführt, die rekombinationsfähig sind (durch red-α, red-β und red-γ) und das MCMV-BAC Plasmid tragen. Durch Doppel-Rekombination wird das PCR-Fragment in das MCMV-BAC Plasmid entsprechend der gewählten Homologien inseriert. Auf E. coli Bakterien mit erfolgreich mutagenisierten MCMV-BAC Plasmid kann mit Chloramphenicol (für BAC) und Kanamycin selektiert werden.

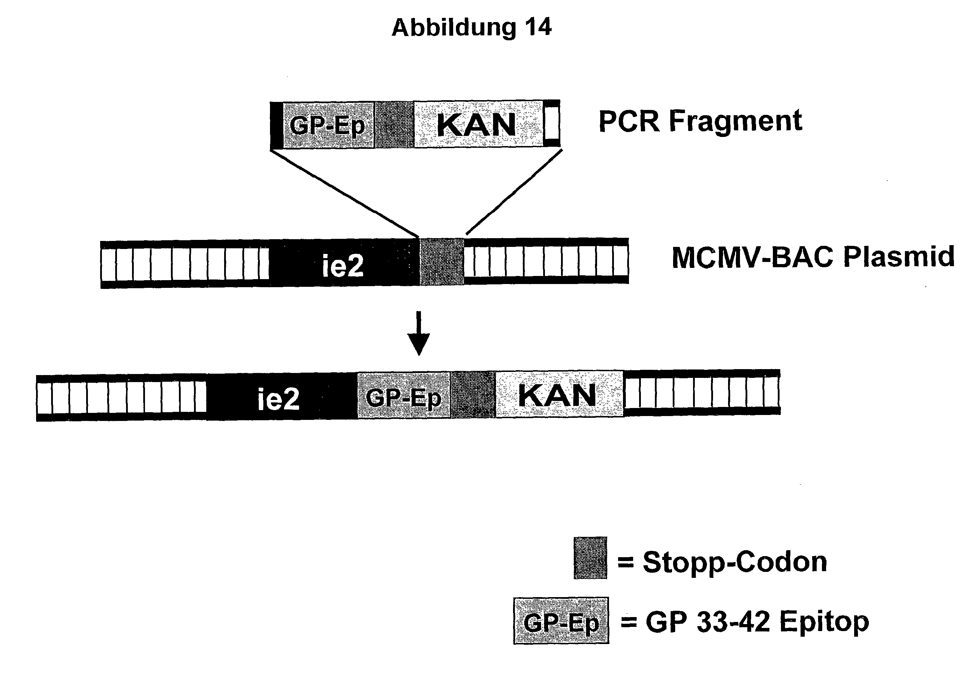

Abb. 14: Schema, wie das GP 33-42 Epitop aus LCMV an das ie2-Gen von MCMV fusioniert wird
   Ein PCR-Fragment, das Homologien auf- und abwärts zum Stopp-Codon des ie2-Gens, das GP 33-42 Epitop als DNA-Sequenz gefolgt von einem Stopp-Codon und das Kanamycin-Gen trägt wird mittels der beschriebenen Verfahren in das MCMV-BAC Plasmid eingeführt. Dadurch entsteht ein MCMV-BAC Plasmid, das ein Fusionsgen von ie2 und GP 33-42 Epitop aus LCMV trägt (ie2-LCMV-GP BAC).

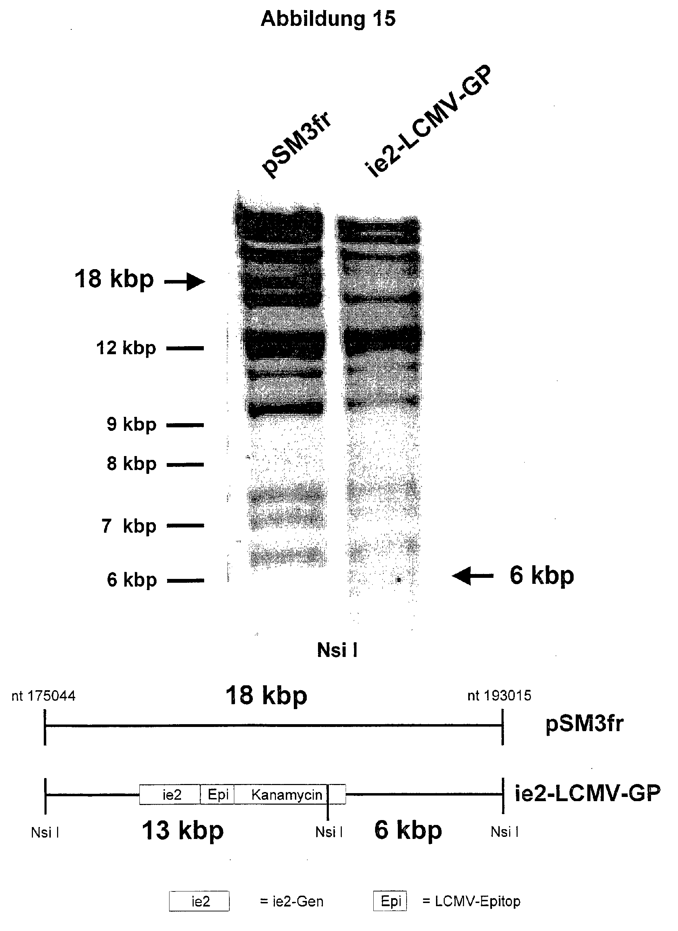

Abb. 15: Restriktionsanalyse des MCMV-BAC mit der Fusion eines LCMV-GP-Epitops an das ie2-Gen von MCMV
   Verdau mit der Restriktionendonuklease NsiI und Auftrennung der entstandenen DNA-Fragmente im Agarosegel zeigt den Verlust einer 18 kbp-Bande und zusätzliche Banden bei 6 und 13 kbp bei dem mutagenisierten ie2-LCMV-GP BAC Plasmid im Vergleich zu dem Wildtyp MCMV-Bac Plasmid(pSM3fr). Unten ist schematisch die Genomorganisation im Bereich der Insertionsstelle des Epitops im MCMV-Genom mit den entsprechenden Nukleotidpositionen (nt) und Restriktionsschnittstellen (NsiI) angegeben.

### BEISPIELE

Wird in den Beispielen Bezug auf die Literaturstelle "Maniatis" genommen, so ist darunter "Maniatis et al., Molecular Cloning: A Laboratory Manual (2^{nd} Edition) (1989), Cold Spring Harbour Laboratory, ISBN 0879693096" zu verstehen.

### Ausführungsbeispiel 1: Verfahren zur Identifizierung von Genen des murinen Cytomegalovirus, die speziell für die Virusvermehrung in Endothel-Zellen wichtig sind

### Kultivierung von Maus-Cytomegalovirus (MCMV) und eukaryontischen Zellen

MCMV (Stamm Smith, ATCC VR-194, ATCC, Rockville, MD, USA) und die davon abgeleiteten Virusmutanten wurden auf NIH-T3-Fibroblasten (ATCC CRL1658) oder SVEC4-10-Endothelzellen (ATCC CRL-2181) nach bekannten Standardprotokollen gezüchtet (Ebeling, A., Keil, G.M., Knust, E., and Koszinowski U.H., J. Virol. (1983) 47, 421-433; Thale, R., Szepan, U., Hengel, H., Geginat, G., Lucin, P., and Koszinowski, U.H., J. Virol. (1995), 69, 6098-6105).

### Plasmidklonierung und Isolierung von Plasmiden und BACs

Plasmidklonierungen wurden nach bekannten Standardprotokollen durchgeführt (Maniatis). Restriktionsendonukleasen wurden von der Firma New England Biolabs (Bevety MA, USA) bezogen. Plasmide und BACs wurden durch Elektroporation in E. coli eingeführt (Maniatis, Shizuya et al., Proc. Natl. Acad. Sci. USA (1992), 89, 8794-8797). Plasmide und BACs wurden nach bekannten Standardprotokollen aus E. coli isoliert (Maniatis; Shizuya et al., Proc. Natl. Acad. Sci. USA (1992), 89, 8794-8797); Messerle et al., Proc. Natl. Acad. Sci. USA (1997), 94, 14759-14763).

### Herstellung des Transposon-Plasmids pTsTM16, welches das Gen für das grün fluoreszierende Protein (GFP) trägt

Das Transposon-Plasmid pTsTM8 (Brune et al., Nature Biotechnology (1999), 17, 360-364) wurde partiell mit der Restriktionsendonuklease BglII verdaut, zum Glätten der DNA-Enden wurde der Verdau mit Klenow-Polymerase behandelt und anschließend zum neuen Plasmid pTsTM8B religiert. Dadurch wurde eine der zwei im Plasmid pTsTM8 vorhandenen BglII-Sequenzen beseitigt. Durch anschließenden Verdau des Plasmids pTsTM8 mit der Restriktionsendonuklease BglII und Ligation mit einem DNA-Fragment, das das GFP-Gen trägt und aus dem Plasmid pEGFP-Cl (der Firma Clonetech) gewonnen wurde, enstand das Transposon-Plasmid pTsTM16.Dieses Transposon-Plasmid ermöglicht die Herstellung von mutanten MCMV-BAC Genomen, die nach Rekonstitution zu lebendem Virus rekombinante Viren entstehen lassen, die das GFP expremieren und dadurch optisch nachgewiesen werden können.

### Transposon-Mutagenese des MCMV-BAC Plasmids mit dem Transposon-Plasmid pTsTM16 zur Herstellung einer Sammlung von mutagenisierten MCMV-BAC Plasmiden in E. coli

Die Transposon-Mutagenese des MCMV-BAC Plasmids pSM3fr, das das gesamte MCMV-Genom trägt (Wagner et al., J. Virol. (1999), 73(8), 7056-7060), mit dem Transposon-Plasmid pTsTM16 in E. coli wurde mit einem Verfahren durchgeführt, das bereits früher beschrieben wurde (Brune et al., Nature Biotechnology (1999), 17, 360-364; PCT/EP98/04816) Das Transposon-Plasmid pTsTM16 wurde durch Elektroporation (Maniatis) in E. coli eingebracht, die bereits das MCMV-BAC Plasmid pSM3fr trugen (siehe Abb. 1). Durch Kultivierung der E. coli in LB-Medium für mehrere Stunden (z.B. über Nacht) bei der permissiven Temperatur von 30°C, die zur Vermehrung des Plasmids pTsTM16 notwendig ist, wurde die Transposition (das "Springen") des Transposons in das MCMV-BAC Genom an beliebiger Stelle ermöglicht. Danach wurden 100 *µ*l der Kultur zum Animpfen von 500 ml LB-Medium verwendet. Dieser Ansatz wurde daraufhin bei der nicht-permissiven Temperatur von 43°C inkubiert. Bei dieser Temperatur kann das Transposon-Plasmid pTsTM16 nicht mehr replizieren. Durch Selektion mit Chloramphenicol und Kanamycin kann auf das Vorhandensein von E. coli selektiert werden, die das MCMV-BAC Plasmid (mit Chloramphenicol-Resistenz) und das in das MCMV-BAC inserierte Transposon (mit Kanamycin-Resistenz) tragen (siehe Abb. 1). Da das Transposon pTsTM16 keine bevorzugte Insertionsstelle für DNA besitzt, wurde damit eine heterogene Sammlung an mutierten MCMV-BAC Plasmiden hergestellt.

### Übertragung der invasiven Eigenschaften auf E. coli Bakterien. die durch Transposon-Mutagenese mutierte MCMV-BAC Plasmide besitzen, durch Einführen des Plasmids pGB2Ωinv-hly

Der bei 43°C inkubierte 500 ml Bakterien-Ansatz mit den durch Transposon-Insertionen mutierten MCMV-BAC Plasmiden wurde bei Erreichen einer optischen Dichte (OD) von 0,6 nach Standard-Protokoll (Maniatis) elektrokompetent gemacht. Anschließend wurde das Invasionsplasmid pGB2Ωinv-hly (Grillot-Courvalin et al., Nature Biotechnology (1998), 16, 862-866) durch Elektroporation (Maniatis) in die elektrokompetenten E. coli eingebracht. Nach Inkubation bei 43°C für eine Stunde und Selektion mit Spectinomycin wurde dieser Transformationsansatz auf LB-Platten, die Spectinomycin, Chloramphenicol und Kanamycin enthalten, ausplattiert und über Nacht bei 43°C inkubiert um einzelne bakterielle Klone zu erhalten (siehe Abb. 1).

### Direkte Virusrekonstitution einer Vielzahl von mutagenisierten MCMV-BAC Plasmiden, die in den invasiv gemachten E. coli enthalten sind

Je ein Loch einer 96-Loch-Mikrotiterplatte (mit 100 *µ*l LB-Medium aufgefüllt) wurde mit einer E. coli Kolonie des auf einer LB-Platte ausplattierten Transformationsansatzes (siehe oben) angeimpft (siehe Abb. 2-A).

Insgesamt wurden 6 Mikrotiterplatten verwendet, das entspricht 576 E. coli Kolonien. Nach Inkubation über Nacht bei 37°C wurden je 2 *µ*l der Bakterienkulturen zum Animpfen von eukaryontischen Maus-Fibroblasten (NIH-3T3), die in einer 96-Loch-Mikrotiterplatte unter Standardbedingungen, aber ohne Antibiotika kultiviert wurden, verwendet (siehe Abb. 2-B). Das Mengenverhältnis von Fibroblastenzelle zu invasiven E. coli Bakterien beträgt durschschnittlich etwa 1 zu 300. Nach zweistündiger Inkubation unter normalen Zellkulturbedingungen wurde das Zellkultur-Medium durch neues Medium ersetzt, welches Ampicillin und Gentamicin in einer Konzentration von jeweils 100 *µ*g/ml enthält.

Durch Aufnahme der E. coli in die Fibroblasten-Zellen und Freisetzung der mutierten MCMV-BAC Genome aus E. coli und den Einschlußvakuolen unter Hilfe der inv- und hly-Genprodukte, kommt es zur Vermehrung von MCMV-Mutanten und nach ca. 3-6 Tagen entstehen erste Virusplaques, falls die in das MCMV-BAC eingefügte Mutation keine für die Virusvermehrung essentielle Genfunktion zerstört hat. Erfolgreiche Vermehrung von lebensfähigen Mutanten kann anhand der Expression des GFP-Gens, das auf dem mutagenisierten viralen Genom vorhanden ist, und der daraus resultierenden Fluoreszenz unter einem Fluoreszenz-Mikroskop optisch kontrolliert werden. Nur erfolgreich mutagenisierte MCMV-Mutanten exprimieren das GFP vom bei der Mutagenese inserierten GFP-Gen aus. Wildtyp MCMV besitzt kein GFP und kann im Fluoreszenz-Mikroskop nicht sichtbar gemacht werden.

Von den 576 E. coli Klonen die auf Fibroblasten überführt wurden, konnten 199 mutante Viren gewonnen werden. (siehe Abb. 2-B).

### Suche eines potentiellen Gens, das für die Vermehrung von MCMV auf Endothelzellen verantwortlich sein könnte

Um ein potentielles virales Gen zu identifizieren, welches die Fähigkeit von MCMV in Endothelzellen effektiv zu replizieren vermittelt, kann man jedes Gen von MCMV separat unterbrechen bzw. entfernen und die Funktion dadurch verhindern. Da die gezielte Mutagenese eines einzelnen viralen Gens innerhalb des MCMV BAC Plasmids sehr arbeits- und zeitaufwendig sein kann, ermöglicht die phänotypische Untersuchung einer großen Zahl von durch Transposonmutagenese gewonnener Virusmutanten, die zufällig ein einzelnes Gen durch Transposoninsertion unterbrochen haben, eine viel schnellere und weniger aufwendige Suche nach einem solchen Gen. Bisher war aber für die Rekonstitution von durch Transposonmutagenese hergestellter mutagenisierter MCMV-BAC Genomen zu Viren die Vermehrung dieser Plasmide, deren Isolierung aus E. coli in größerem Maßstab und die Transfektion dieser DNA in Fibroblasten ein sehr zeit- und kostenintensiver Vorgang. Die Rekonstitution von mehreren hundert Visusmutanten wäre somit nur mit sehr großem Aufwand möglich gewesen. Die direkte Rekonstitution der Virusmutanten aus den E. coli Bakterien ohne DNA-Isolierung und Transfektion der Fibroblasten, die diese Patentanmeldung beschreibt, ermöglicht nun die schnelle und ökonomische Untersuchung vieler Mutanten.

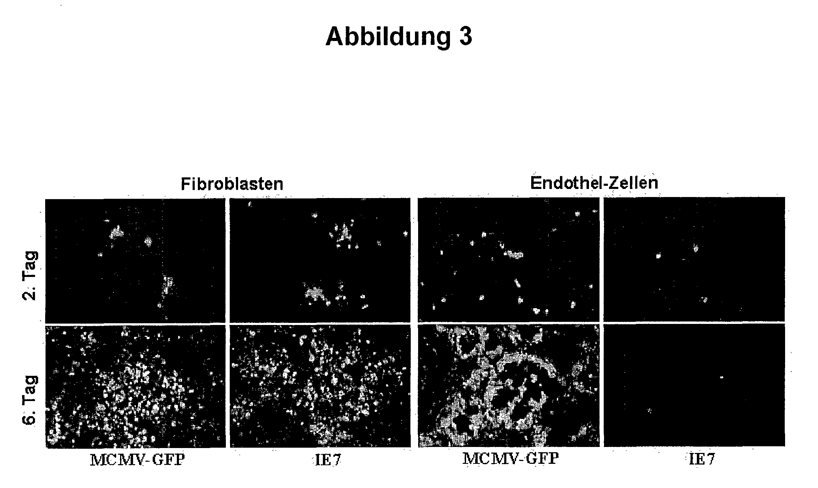

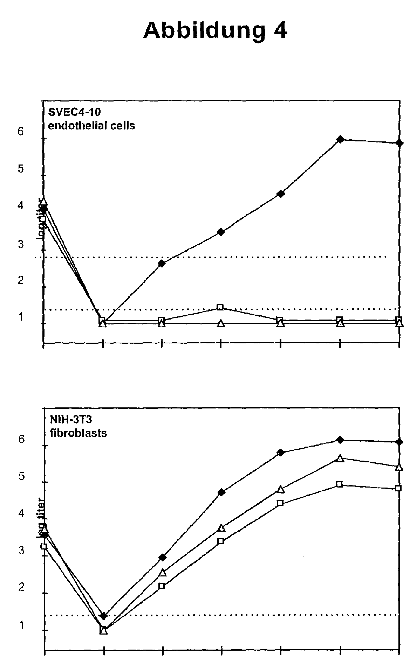

Der Zellkulturüberstand von den Zellen, die mit diesen 199 rekonstituierten Virus-Mutanten infiziert waren, wurde auf neue Fibroblastenkulturen in einer 24-Loch-Kulturplatte zur weiteren Virusvermehrung überführt (siehe Abb. 2-C). Nach wenigen Tagen waren die NIH-3T3 Zellen durch die Virusinfektion lysiert. Je 200 *µ*l der Zellkulturmedium-Überstände dieser lysierten Zellen wurden jeweils auf NIH-3T3-Zellen und parallel auf SVEC4-10-Endothelzellen gegeben (siehe Abb. 2-D). 6 Tage nach der Infektion mit diesen Virusmutanten wurde die Virusvermehrung in diesen Zellen bestimmt: Optisch durch Betrachtung der grünen fluoreszierenden Zellen (siehe Abb 3 zum Vergleich einer ausgewählten Virusmutante [IE7] mit MCMV Wildtyp 2 und 6 Tage nach Infektion) und durch Virustiterbestimmung. Für die Titerbestimmung wurden die Zellkulturmedium-Überstände geerntet und die darin enthaltenden Virusmengen nach der Standard-Titrationsmethode TCID₅₀ bestimmt. In Abb. 4 sind die Virustiter von 2 exemplarisch ausgewählten Virusmutanten im Verlauf der Infektion von Tag 0 bis Tag 6 nach Infektion auf NIH-3T3- und SVEC4-10-Zellen dargestellt. Im Vergleich zu Wildtyp-MCMV zeigen die beiden Mutanten in NIH-3T3-Zellen vergleichbare Virustiter, auf den SVEC4-10-Zellen lagen die Titer unter dem Detektionsniveau (gestrichelte Linie).

Es wurden insgesamt 6 Virusmutanten identifiziert die sich zwar auf NIH-3T3-Zellen, aber nicht auf den Endothelzellen vermehren können. Für diese 6 Virusmutanten wurde die entsprechende MCMV-BAC DNA aus E. coli isoliert (nach Standardmethode, siehe Maniatis bzw. Messerle et al., Proc. Natl. Acad. Sci. USA (1997), 94, 14759-14763) und mit einem Primer, der innerhalb des inserierten Transposons bindet und in die virale Sequenz hinein liest, sequenziert (Methode siehe Brune et al., Nature Biotechnology (1999), 17, 360-364).

Hierbei zeigte sich, daß alle 6 Virusmutanten auf einer Transposoninsertion in die Gene M45 und m45.1 beruhen (siehe Abb. 5). Zudem zeigte die Sequenzierung dieses gesamten Genombereiches im ursprünglichen Wildtyp-Genom, daß es sich entgegen der publizierten MCMV-Sequenz nicht um zwei Gene (M45 und m45.1) handelt, sondern um einen einzigen offenen Leserahmen (ORF). Somit konnte ein spezifisch für die Vermehrung von MCMV auf Endothelzellen verantwortliches Gen identifiziert werden.

### Ausführungsbeispiel 2: Immunisierung von Mäusen (Stamm G129) mit einem BAC, das das MCMV-Genom und zusätzlich das HBs-Antigen (MCMV-HBs BAC) trägt, mittels invasiven E. coli Bakterien als Überträger des MCMV-HBs BAC

### A) Hintergrund und Ziel der Immunisierungsversuche

Lebendimpfstoffe von Herpesviren sind aufwendig zu produzieren und transportieren. Die Viren müssen auf Säugetierzellen angezüchtet werden und können nur gekühlt gelagert und transportiert werden, um eine Inaktivierung zu vermeiden. So gibt es beispielsweise gegen herpesvirale Infektionen nur zwei wirksame und zugelassene Impfstoffe. Hierbei handelt es sich um attenuierte Varizella-Zoster-Viren bzw. Pseudorabies-Viren. Ein neues Verfahren das es erlaubt, einen Impfstoff einfach zu produzieren, zu lagern und zu verabreichen, ist daher wünschenswert.

Die Verwendung von herpesviralen BAC-Genomen und Methoden zur Veränderung der darin enthaltenen DNA erlaubt die einfache und schnelle Herstellung von abgeschwächten Virus-Varianten. Zusätzlich können Fremdgene oder Fremdepitope in das Genom eingeführt werden, um gleichzeitig eine Immunisierung gegen mehrere Erreger unterschiedlicher Art zu erzielen. Da die herpesviralen BAC-Plasmide eine infektiöse DNA enthalten können - d.h. die Einbringung der BAC-DNA in Wirtszellen führt zur Produktion von Virus in diesen Zellen - kann mit diesen herpesviralen BAC-Plasmiden eine DNA-Vakzine hergestellt werden, die zur selbsständigen Vermehrung der Antigene führt, aber keine Anzüchtung von Viren erfordert. Die virale DNA muß in die Zellen des zu impfenden Organismus eingebracht werden. Bisher bekannte Methoden zur Einbringung des viralen BAC-Plasmids in die Wirtszellen sind zum Beispiel Injektionen der DNA in die Haut oder Einschuß BAC-beschichteter Goldpartikel in die Zellen der Haut. Für HSV-1 wurde die Durchführbarkeit eines solchen Ansatzes gezeigt (Suter et al., Proc. Natl. Acad. Sci. USA (1999), 96(22), 12697-12707).

In diesem Anwendungsbeispiel wird an einem Maus-Modell gezeigt, daß eine Verabreichung eines viralen BAC-Genoms durch Bakterien als Überträger möglich ist (siehe Prinzip in Abb. 6). Die verwendeten E. coli Bakterien, die das MCMV-HBs BAC Plasmid (es besitzt das gesamte MCMV Genom und zusätzlich das HBs-Gen des Hepatitis B-Virus) und das Invasionsplasmid pGB2Ωinv-hly tragen, können auf unterschiedlichem Weg verabreicht werden: intraperitoneal, subkutan, intramuskulär oder über die Schleimhäute (also auch durch einfaches Schlucken = per os).

### B) Kultivierung von Maus-Cytomegalovirus (MCMV) und eukaryontischen Zellen

MCMV (Stamm Smith, ATCC VR-194, ATCC, Rockville, MD, USA) und die Virusmutante RV MCMV-HBs wurden auf embryonalen Balb/c-Maus-Fibroblasten (MEF) und NIH-T3-Fibroblasten (ATCC CRL1658) nach bekannten Standardprotokollen gezüchtet (Ebeling, A., Keil, G.M., Knust, E., und Koszinowski U.H., J. Virol. (1983), 47, 421-433; Thäle, R., Szepan, U., Hengel, H., Geginat, G., Lucin, P., und Koszinowski, U.H., J. Virol. (1995), 69, 6098-6105).

### C) Tiere und Infektionsbedingungen

Bei allen Versuchen wurden Mäuse des Stamms G129 (IFNγR^{-/-}) verwendet (van den Broek M.F., Müller U., Huang S., Zinkernagel R.M., and Aguet M., Immunol. Rev. (1995), 148, 5-18). Infektionsbedingungen für alle Versuche wurden unter etablierten Standardbedingungen durchgeführt, die bereits beschrieben wurden (Jonjic, S., Pavic, I., Polic, B., Crnkovic, I., Lucin, P., und Koszinowski, U.H., J. Exp. Med. (1994), 179, 1713-1717 und Reddehase, M.J., Keil, G.M., und Koszinowski, U.H., J. Immunol. (1984), 132, 482-489).

### D) Plasmidklonierung und Isolierung von Plasmiden und BACs

Plasmidklonierungen wurden nach bekannten Standardprotokollen durchgeführt (Maniatis). Restriktionsendonukleasen wurden von der Firma New England Biolabs (Bevety MA, USA) bezogen. DNA wurde in E. coli (DH10B) durch Elektroporation mit dem "Gene Pulser"-Gerät von BioRad (2.5 kV, 25 *µ*F, 400 Ohm) eingebracht. Transformanten wurden auf LB-Platten (mit entsprechenden Standard-Antibitika-Konzentrationen; für BAC Plasmide: 12,5 *µ*g/ml Chloramphenicol bzw. 25 *µ*g/ml Kanamycin) selektiert (Maniatis; Messerle et al., Proc. Natl. Acad. Sci. USA (1997), 94, 14759-14763). High-Copy-Plasmide und BAC Plasmide wurden nach bekannten Standardprotokollen durch alkalische Lyse aus E. coli isoliert (Maniatis; Shizuya et al., Proc. Natl. Acad. Sci. USA (1992), 89, 8794-8797; Messerle et al., Proc. Natl. Acad. Sci. USA (1997), 94, 14759-14763). Zur Charakterisierung der MCMV-BAC-DNA wurde Virus durch Transfektion von MEF mit dem mutagenisierten MCMV-BAC Plasmid (1 *µ*g) rekonstituiert. Dies erfolgte durch Calcium Phosphat Präzipitation entsprechend Standardprotokollen (Thäle, R., Szepan, U., Hengel, H., Geginat, G., Lucin, P., und Koszinowski, U.H., J. Virol. (1995), 69, 6098-6105 und Messerle, M., Bühler, B., Keil, G.M., und Koszinowski, U.H., J. Virol. (1992) 66, 27-36). Zur Charakterisierung der MCMV-DNA aus rekonstituiertem Virus wurde die gesamte zelluläre DNA aus Zellen isoliert. Die Zellen wurden durch Behandlung mit Trypsin und 5 minütiger Zentrifugation bei 800 g geerntet und mit 50 mM Tris-HCl (pH 8), 20 mM EDTA, 0,5 % SDS und 0,5 mg/ml Proteinase K lysiert. Nach Inkubation dieses Ansatzes für 3 Stunden bei 56°C wurde die DNA zweimal Phenol/Chloroform (1:1) extrahiert und mit zwei Volumen 100% Ethanol präzipitiert (Ebeling, A., Keil, G.M., Knust, E., und Koszinowski U.H., J. Virol. (1983), 47, 421-433; Mercer, J.A., Marks, J.R., und Spector, D.H., Virology (1983), 129, 94-106).

Zur Analyse wurde die DNA mit Restriktionsendonukleasen verdaut und die entstandenen DNA-Fragmente durch Agarosegel-Elektrophorese (0,8 % Gel) aufgetrennt, wie schon beschrieben in Ebeling et al., 1983 (a.a.O.).

### E) Herstellung eines MCMV-BAC Plasmids, das das HBs-Antigen des humanen Hepatitis B Virus exprimiert

Das S-Gen des humanen Hepatitis B-Virus (HBs) wurde durch Restriktionsendonuklease-Verdau mit XhoI und NsiI aus dem Plasmid pMH3/3091 (Junker et al., Nucleic Acids Res. (1987), 15(24), 10117-10132) herausgeschnitten und in das Plasmid pBluescriptKSII+ (Stratagene, La Jolla, CA) einkloniert. Von diesem Plasmid wurde HBs durch Restriktionsendonuklease-Verdau mit KpnI und NotI erneut ausgeschnitten und in das mit KpnI und NotI behandelte Plasmid pRep4 (Invitrogen, Carlsbad, CA, USA) einkloniert. Die dadurch entstandene Expressionskassette (mit einem RSV-Promotor, dem HBs und dem Polyadenylierungssignal von SV40) wurde mit der Restriktionsendonuklease SalI aus dem Plasmid herausgeschnitten und nach Behandlung mit der Klenow-Polymerase zum Glätten der DNA-Enden in das (in dem Plasmid pACYC177 einklonierte) HindIII-L-Fragment des MCMV Genoms (Ebeling et al., J. Virol. (1983), 47, 421-433) zwischen den beiden Restriktionsschnittstellen HpaI (Nukleotid-Position 184236 und 184315) eingefügt. Aus diesem Plasmid wurde das HindIII-L-Fragment mit der darin einklonierten Expressionskassette (siehe Oben) mit HindIII ausgeschnitten und in das Plasmid pMB096 (O'Conner et al., Science (1989), 244, 1307-1312) einkloniert. Zum Einfügen des HindIII-L-Fragmentes mit der Expressionskassette in das MCMV-BAC Plasmid pSM3fr (Wagner et al., J. Virol. (1999), 73(8), 7056-7060) wurde die Rekombinationsmethode von Messerle et al., Proc. Natl. Acad. Sci. USA (1997), 94, 14759-14763 verwendet. Dadurch enstand das MCMV-HBs BAC Plasmid. Zur Kontrolle einer korrekten Mutagenese wurde das MCMV-HBs BAC Plasmid und das Wildtyp-BAC Plasmid pSM3fr aus E. coli isoliert und zudem rekombinante Virus-DNA von rekonstituierten Viren aus Zellen isoliert. Diese DNA wurde mittels Restriktionsendonuklease-Verdau mit HindIII analysiert. Das Verschieben des HindIII-L-Fragmentes im MCMV-HBs BAC Plasmid bzw. im rekombinanten Virus RV MCMV-HBs von 7,5 kbp zu 9,7 kbp im Vergleich zu pSM3fr mit dem Wildtyp-MCMV-Genom bestätigte die korrekte Insertion des HBs in das MCMV Genom (siehe Abb. 7).

### F) Intraperitoneale Verabreichung der MCMV-HBs BAC tragenden Bakterien in immunsuppremierten Mäusen führt zur MCMV-Vermehrung und Expression des HBs-Proteins

5 Mäuse (Stamm G129, IFNγR^{-/-}) bekamen eine Dosis von 10^{**8**} E. coli Bakterien (die das MCMV-HBs BAC und das Plasmid pGB2Ωinv-hly besitzen und über Nacht in LB-Medium versetzt mit 50 *µ*g/ml Spectinomycin und 12,5 *µ*g/ml Chloramphenicol bei 37°C kultiviert wurden) intraperitoneal verabreicht. Hierfür wurde die LB-Bakterien-Lösung 1:10 mit PBS verdünnt und 500 *µ*l dieser Lösung gespritzt. Als Kontrolle wurden E. coli Bakterien verwendet, die nur das Plasmid pREP4-HBs (HBs-Expressionskassette im Plasmid pREP4 [von der Firma Invitrogen]), welches das gleiche HBs-Gen enthält aber kein MCMV-BAC, besitzen. Um zu testen, ob die Bakterien das virale BAC-Genom übertragen haben, eine MCMV-Infektion stattgefunden hat (falls dies der Fall war besitzen die Mäuse nun latentes MCMV, welches durch Immunsuppression reaktiviert werden kann) und ob das heterologe HBs-Gen exprimiert wurde, wurden die Mäuse nach Standardprotokollen (Jonjic, S., Pavic, I., Polic, B., Crnkovic, I., Lucin, P., und Koszinowski, U.H., J. Exp. Med. (1994), 179, 1713-1717) immunsuppressiv behandelt. Jeweils 100 *µ*g Antikörper gegen Maus-CD4- und Maus-CD8-Oberflächenmoleküle (gewonnen aus der Ratte) wurden den Mäusen am 6. und 13. Tag nach der primären Infektion verabreicht. Zudem wurden den Mäusen ab dem 13. Tag nach der Infektion täglich intramuskulär 6,25 *µ*g Hydrocortison verabreicht. Am 20.Tag nach der Infektion wurden aus den Schwanz-Venen jeweils 100 *µ*l Serum entnommen und die HBs-Antigen-Titer mit einem ELISA HBs-Antigen-Nachweis (ORTHO) bestimmt. Im Serum aller Mäuse konnte signifikante Konzentrationen an HBs-Antigen nachgewiesen werden, was die effiziente Expression des im MCMV-BAC inserierten HBs-Gens aus dem Hepatitis B-Virus beweist (siehe Abb. 8).

Zudem wurden die Mäuse am 21. Tag nach der Infektion getötet und die vorhandenen Virusmengen in verschiedenen Organen durch Standard-Plaque-Assays (Reddehase, M.J., Keil, G.M., und Koszinowski, U.H., J. Immunol. (1984), 132, 482-489 und Reddehase, M.J., Weiland, F., Münch, K., Jonjic, S., Lüske, A., und Koszinowski, U.H., J. Virol. (1985), 55, 264-273) bestimmt. In allen 5 Mäusen konnte nach Immunsuppression infektiöses rekombinantes MCMV nachgewiesen werden (siehe Abb. 9).

### G) Intraperitoneale Verabreichung der MCMV-HBs BAC tragenden Bakterien in Mäuse führt zur Bildung von Antikörpern gegen MCMV

5 Mäuse (Stamm G129, IFNγR^{-/-}) bekamen eine Dosis von 10^{**8**} E. coli Bakterien (die das MCMV-HBs BAC und das Plasmid pGB2Ωinv-hly besitzen und über Nacht in LB-Medium versetzt mit 50 *µ*g/ml Spectinomycin und 12,5 *µ*g/ml Chloramphenicol bei 37°C kultiviert wurden) intraperitoneal gespritzt. Hierfür wurde die LB-Bakterien-Lösung 1:10 mit PBS verdünnt und 500 *µ*l dieser Lösung gespritzt. 42 Tage und 60 Tage nach der Verabreichung der Bakterien wurden jeder der 5 Mäuse aus der Schwanzvene 200 *µ*l Serum entnommen. Dies wurde 30 Minuten bei Raumtemperatur inkubiert, anschließend 4 Minuten bei 4000 rpm zentrifugiert und bei -20°C gelagert um gleichzeitig Antikörpertiter gegen MCMV im ELISA-Test (Beschreibung des Tests siehe bei Jonjic, S., Del Val, M., Keil, G.M., Reddehase, M.J., und Koszinowski, U.H., J. Virol. (1988), 62, 1653-1658) zu bestimmen. Hierbei konnten in allen 5 Mäusen signifikante Megen an Antikörpern gegen MCMV nachgewiesen werden.

### H) Verabreichung der MCMV-HBs BAC tragenden Bakterien in Mäuse über die Schleimhäute (per os) führt zur Bildung von Antikörpern gegen MCMV

11 Mäuse (Stamm G129, IFNγR^{-/-}) bekamen E. coli Bakterien, die das MCMV-HBs BAC und das Plasmid pGB2Ωinv-hly besitzen und über Nacht in LB-Medium versetzt mit 50 *µ*g/ml Spectinomycin und 12,5 *µ*g/ml Chloramphenicol bei 37°C kultiviert wurden, verabreicht. Im Detail: Die Bakterienlösung wurde 8 Minuten bei 4°C abzentrifugiert und mit PBS verdünnt. Die Endkonzentrationen der Bakterien betrugen 2x10⁸ Bakterien/ml PBS für die intraperitoneale Kontroll-Verabreichung und 2x10¹⁰ Bakterien/ml PBS für die Verabreichnung per os (über die Schleimhäute).

Für die Verabreichnung per os wurden leicht betäubten Mäusen 350 *µ*l der PBS-Bakterienlösung (entspricht ca. 7x10⁹ Bakterien) mit einer Mikropipette in den Rachen verabreicht.

Für die intraperitoneale Kontroll-Verabreichung wurden 500 *µ*l der Bakterienlösung (entspricht 10⁸ Bakterien) intraperitoneal gespritzt.

3 Wochen nach der Verabreichung der Bakterien wurden jeder Maus 200 *µ*l Serum aus der Schwanzvene entnommen. Der ELISA-Test zur Bestimmung der Antikörpermenge gegen MCMV (zum Testablauf siehe Jonjic, S., Del Val, M., Keil, G.M., Reddehase, M.J., und Koszinowski, U.H., J. Virol. (1988), 62, 1653-1658) zeigte signifikante Antikörpermengen bei einer von 11 per os infizierten Mäuse, was die grundsätzliche Durchführbarkeit der Impfung über die Schleimhäute von Mäusen mit MCMV-BAC enthaltenden E. coli beweist. In den peritoneal infizierten Mäusen zeigten sich signifikante Antikörper-Konzentrationen bei 8 von 11 Mäusen.

### I) Verabreichung der MCMV-HBs BAC tragenden Bakterien in Mäuse durch Injektion unter die Haut und durch intramuskuläre Injektion führt zu Antikörperbildung gegen MCMV

Jeweils Gruppen von 5 Mäusen (Stamm G129, IFNγR^{-/-}) wurden intramuskulär oder unter die Haut (subkutan) mit E, coli Bakterien infiziert. Die E. coli trugen
a) das MCMV-HBs-BAC und das Invasionsplasmid pGB2Ωinv-hly oder
b) das Plasmid pREP4-HBs (High-Copy-Plasmid, welches das HBs-Antigen exprimiert) und das Invasionsplasmid pGB2Ωinv-hly.
Mäuse die mit Bakterien injiziert wurden, die die Plasmide wie unter a) beschriebenen besitzen, sollten effektiv Antikörper gegen MCMV bilden.

Die unter b) verwendeten Plasmide in den E. coli Bakterien dienten als Negativ-Kontrolle.

Für die intramuskuläre Verabreichung wurden 100 *µ*l Bakterien in PBS (insgesamt etwa 5x10⁸ Bakterien) in den Muskel der Hinterbeine gespritzt. Für die subkutane Verabreichung wurden ebenfalls 100 *µ*l Bakterien in PBS (insgesamt etwa 5x10⁸ Bakterien) unter die Haut im Nacken injiziert.

28 Tage nach der Verabreichung der Bakterien wurde jeweils 200 *µ*l Serum aus der Schwanzvene entnommen und die Menge an MCMV-spezifischen Antikörpern im ELISA bestimmt (siehe oben).

### I-1) Ergebnisse für die in Mäuse subkutan verabreichten Bakterien mit MCMV-HBs-BAC und Invasionsplasmid pGB2Ωinv-hly:

Wie in Abb. 10-A zu sehen ist, bildeten 3 von 5 Mäusen, die E. coli mit MCMV-HBs-BAC und pGB2Ωinv-hly erhielten, signifikante Antikörpertiter gegen MCMV aus. Rechts in der Abb. 10-A ist der Kontroll-ELISA gezeigt mit unbehandelten Zellen, bei dem es keine signifikanten Signale gab. In Abb. 9-B sind als Negativkontrolle die Antikörpermengen der Mäuse gezeigt, die mit Bakterien, die nur das Kontrollplasmid pREP4-HBs und das Invasionsplasmid pGB2Qinv-hly besaßen, infiziert wurden. Hier ist keine Bildung von MCMV-spezifischen Antikörpern festzustellen.

### I-2) Ergebnisse für die in Mäuse intramuskulär verabreichten Bakterien mit MCMV-HBs-BAC und Invasionsplasmid pGB2Ωinv-hly:

Abb. 11-A zeigt, daß alle 5 Mäuse einen signifikanten Antikörpertiter gegen MCMV ausgebildet haben. Rechts in der Abb. 11-A ist die Negativ-Kontrolle für den ELISA-Test zu sehen. In Abb. 11-B sind als Negativkontrolle die Antikörpermengen der Mäuse gezeigt, die mit Bakterien, die nur das Kontrollplasmid pREP4-HBs und das Invasionsplasmid pGB2Ωinv-hly besaßen, infiziert wurden. Hier ist keine Bildung von MCMV-spezifischen Antikörpern festzustellen.

### J) Verabreichung der MCMV-HBs BAC tragenden Bakterien in Mäuse durch Injektion unter die Haut und durch intramuskuläre Injektion führt zum Schutz gegen eine MCMV-Infektion

Um zu testen, ob die Bildung von spezifischen Antikörpern gegen MCMV nach intramuskulärer und subkutaner Verabreichung der Bakterien (siehe I) auch einen Schutz gegen eine erneute MCMV-Infektion bietet, wurden die unter I) beschriebenen Mäuse 28 Tage nach der Infektion mit einer tödlichen Dosis von 5x10⁴ PFU des in der Speicheldrüse passagierten Wildtyp-MCMV (virulenter als MCMV aus der Zellkultur) intravenös infiziert.

Abb. 12-A zeigt, daß alle Mäuse, die mit Bakterien, die nur das Kontrollplasmid pREP4-HBs und Plasmid pGB2Qinv-hly trugen, auf subkutanen Weg "immunisiert" wurden, nach 8 Tagen tot waren. Im Gegensatz dazu überlebten 4 von 5 Mäusen, die mit Bakterien auf subkutanem Weg infiziert wurden, die das MCMV-HBs-BAC Plasmid und das Plasmid pGB2Ωinv-hly trugen. Durch Übertragung des MCMV-HBs-BAC Plasmids mittels invasiver Bakterien konnten also nicht nur Antikörper gegen MCMV induziert werden, sondern auf diesem Wege immunisierte Mäuse waren tatsächlich vor einer MCMV-Infektion geschützt.

Die gleichen Ergebnisse ergaben sich für die auf intramuskulärem Weg immunisierten Mäuse. Hierbei überlebten alle Mäuse die MCMV-Infektion, die über die E. coli Bakterien das MCMV-HBs-BAC Plasmid (mit dem Invasionsplasmid pGB2Ωinv-hly) erhalten haben. Von den mit nur dem Kontrollplasmid pREP4-HBs immunisierten Mäusen waren alle nach 8 Tagen tot (siehe Abb. 12-B).

### Ausführungsbeispiel 3: Direkte Fusion der Epitopsequenz 5'-aaggctgtctacaattttgccacctgt aus Lymphocytic choriomeningitis Virus (LCMV) an das C-terminale Ende des ie2-Gen von MCMV

### Einführung

Eine schnelle Mutagenesemethode für DNA stellt die Doppel-Rekombination mit einem linearen DNA-Fragment (das die mutierte DNA-Sequenz und flankierende homologe Sequenzen von ca. 50 bp trägt) vermittelt durch das ET-Rekombinationssystem dar (PCT/EP98/07945). Obwohl diese Methode die Herstellung einer Deletionsmutante in wenigen Tagen und Nukleotid-genau ermöglicht, benötigt das Inserieren von Fremd-DNA (Epitopen, Genen, etc.) oder das Einführen von Punktmutationen mit dieser Methode eine zweite Mutagenese-Runde. Die notwendige Gegenselektion gegen einen Negativ-Marker (z.B. SacB) (PCT/EP98/07945) ist zudem sehr ineffizient und für manche virale Genome, die viele interne Sequenzwiederholungen besitzen (wie z.B. MCMV), aufgrund von Instabilität des Genoms nicht anwendbar.

Die Erfindung ermöglicht nun u.a. auch die Nukleotid-genaue Insertion von kurzen DNA-Sequenzen (wie z.B. Epitopen und Erkennungssequenzen wie FlagTag odere HA-Tag) im richtigen Leserahmen ("In-Frame") mit einer einzigen Transformation und ohne Sub-Klonierung von viralen DNA-Sequenzen (Abb. 13). Die Methode beruht auf einem doppelten Rekombinationschritt eines linearen DNA-Fragments (hergestellt durch PCR) mit dem Virus-DNA enthaltenden BAC in DH10B-Bakterien. Diese Bakterien sind recA- (erhöht die Stabilität des BAC in E. coli) und tragen neben dem gewünschten BAC auch das pBADαβγ Plasmid (Zhang et al., Nat. Genet. (1998), 20(2), 123-128). In diesem Anwendungsbeispiel wird nun die in das virale Genom zu inserierende Epitop-Sequenz zusätzlich in das synthetische Oligonukleotid, das für die PCR verwendet wird, integriert. Die Rekombinationsproteine redα, redβ (ermöglichen die Rekombionation von nur 35-55 bp-großen DNA-Homologien) und redγ (hemmt Exonukleaseaktivität und damit Abbau von linearer DNA) werden in den DH10B-Bakterium kontrolliert expremiert (Zhang et al., Nat. Genet. (1998), 20(2), 123-128). Das in die Bakterien durch Transformation überführte DNA-Fragment wird durch eine einzige Polymerasekettenreaktion (PCR) hergestellt. Die hierbei verwendeten synthetischen Primer sind aussergewöhnlich lange und erfordern besondere PCR-Bedingungen (Touch-Down-PCR). Sie tragen nicht nur die zum primären "Priming" notwendigen Homologien zu einem Markergen (hier Kn-Kassette), sondern auch noch Homologien von 50 Basenpaaren (bp) zu Sequenzen im MCMV-BAC Plasmid. Ein Primer enthält nun zusätzlich die GP33-41-Epitop-Sequenz (Aminosäuresequenz: KAVYNFAT) aus LCMV.

### Kultivierung von Maus-Cytomegalovirus (MCMV) und eukaryontischen Zellen

MCMV (Stamm Smith, ATCC VR-194, ATCC, Rockville, MD, USA) und die davon abgeleiteten Virusmutanten wurden auf NIH-T3-Fibroblasten (ATCC CRL1658) und auf embryonalen Balb/c-Maus-Fibroblasten (MEF) nach bekannten Standardprotokollen gezüchtet (Ebeling, A., Keil, G.M., Knust, E., und Koszinowski U.H., J. Virol. (1983), 47, 421-433; Thale, R., Szepan, U., Hengel, H., Geginat, G., Lucin, P., und Koszinowski, U.H., J. Virol. (1995), 69, 6098-6105).

### Plasmidklonierung, Polymerasekettenreaktion (PCR) und Isolierung von Plasmiden und BACs

Plasmidklonierungen wurden nach bekannten Standardprotokollen durchgeführt (Maniatis). Restriktionsendonukleasen wurden von der Firma New England Biolabs (Bevety MA, USA) bezogen. DNA wurde in E. coli (DH10B) durch Elektroporation mit den "Gene Pulser"-Gerät von BioRad (2.5 kV, 25 *µ*F, 400 Ohm) eingebracht. Transformanten wurden auf LB-Platten (mit entsprechenden Standard-Antibitika-Konzentrationen; für BAC Plasmide: 12,5 *µ*g/ml Chloramphenicol bzw. 25 *µ*g/ml Kanamycin) selektiert (Maniatis; Messerle et al., Proc. Natl. Acad. Sci. USA (1997), 94, 14759-14763). High-Copy-Plasmide und BAC Plasmide wurden nach bekannten Standardprotokollen durch alkalische Lyse aus E. coli isoliert (Maniatis; Shizuya et al., Proc. Natl. Acad. Sci. USA (1992), 89, 8794-8797; Messerle et al., Proc. Natl. Acad. Sci. USA (1997), 94, 14759-14763). Zur Charakterisierung der MCMV-BAC-DNA wurde Virus durch Transfektion von MEF mit dem mutagenisierten MCMV-BAC Plasmid (1 *µ*g) rekonstituiert. Dies erfolgte durch Calcium Phosphat Präzipitation entsprechend Standardprotokollen (Thäle, R., Szepan, U., Hengel, H., Geginat, G., Lucin, P., und Koszinowski, U.H., J. Virol. (1995), 69, 6098-6105 und Messerle, M., Bühler, B., Keil, G.M., und Koszinowski, U.H., J. Virol. (1992), 66, 27-36). Zur Charakterisierung der MCMV-DNA aus rekonstituiertem Virus wurde die gesamte zelluläre DNA aus Zellen isoliert. Die Zellen wurden durch Behandlung mit Trypsin und 5 minütiger Zentrifugation bei 800 g geerntet und mit 50 mM Tris-HCl (pH 8), 20 mM EDTA, 0,5 % SDS und 0,5 mg/ml Proteinase K lysiert. Nach Inkubation dieses Ansatzes für 3 Stunden bei 56°C wurde die DNA zweimal Phenol/Chloroform (1:1) extrahiert und mit zwei Volumen 100% Ethanol präzipitiert (Ebeling, A., Keil, G.M., Knust, E., und Koszinowski U.H., J. Virol. (1983), 47, 421-433; Mercer, J.A., Marks, J.R., and Spector, D.H., Virology (1983), 129, 94-106.).

Zur Analyse wurde die DNA mit Restriktionsendonukleasen verdaut und die entstandenen DNA-Fragmente durch Agarosegel-Elektrophorese (0,8 % Gel) aufgetrennt, wie schon beschrieben in Ebeling et al., 1983 (a.a.O).

Die PCR wurde nach Standardprotokollen durchgefürt (Maniatis, Zhang et al., Nat. Genet. (1998), 20(2), 123-128). Für eine effizientere Ausbeute mit den ungewöhnlich langen Oligonukleotiden wurde Touch-Down PCR angewandt. Dieses Verfahren ist dem Fachmann bekannt.

### Herstellung eines PCR-Produktes, das das Epitop GP 33-41 aus LCMV zwischen einer DNA-Sequenz, die zum ie2-Gen-Ende homolog ist, und dem Kanamycin-Selektionsmarker trägt

Für die PCR zur Herstellung eines linearen DNA-Fragments, das virale Homologien auf- und abwärts des Stopp-Codons des ie2-Gens, die Epitopsequenz GP 33-42 (Nukleotidseuqenz: *aaggctgtctacaattttgccacctgt)* und den Kanamycin-Selektionsmarker trägt (siehe schematische Abb. 14) wurden folgende Primer verwendet:

5`-Primer

Homologie zum viralen Genom

5´-gaaccacgggttctttctcttgaccagagacctggtgaccgtcaggaagaagattcag

Spacer GP 33-42-Epitop-Sequenz Spacer Stopp

ggtatc aaggctgtctacaattttgccacctgt gggata TGA

Priming-Region für Kan-Gen

CGATTTATTC AACAAAGCCA CG

3´-Primer

Homologie zu viralen Genom von Nukleotid 187300 bis 187361

5´-ctgtccgatg aataaaacct ctttatttat tgattaaaaa ccatgacata

Priming-Region für Kan-Gen

cctcgtgtcc tc GCCAGTGT TACAACCAAT TAACC

### Mutagenese des MCMV-BAC Plamsids pSM3fr

Das entstandene PCR-Produkt wurde nach unten angegebenen Protokoll, angelehnt an an Zhang et al., Nat. Genet. (1998), 20(2), 123-128 zur Mutagenese verwendet:

### Protokoll

### Vorraussetzung:

■ Bakterien (DH10B) mit MCMV-BAC Plasmid pSM3fr; diese elektrokompetent machen und das Plasmid pBADaßy durch Elektroporation einführen
■ hierfür Selektion auf LB-Platten mit 100 *µ*g/ml Ampicillin und 25 *µ*g/ml Chloramphenicol

### PCR-Fragment herstellen:

→ normale PCR-Bedingungen laut Protokoll der High-Fidelity-Polymerase von der Firma Roche, aber mit Touch-Down-Bedingungen: 94°C-62°C-68°C, wobei die Annealing-Temperatur von 62°C bei jedem Zyklus um ein °C abnimmt (von 62°C bis 45°C) -18 Zyklen, dann weitere 17 Zyklen bei 45°C
→ als Template-DNA dient das Plasmid pACYC177 (New England Biolabs)
→ nur sehr wenig Template DNA verwenden (3 ng)
→ 100 *µ*l Gesamtvolumen

■ 5 *µ*l Probeauftrag auf Gel, um zu überprüfen, ob die PCR erfolgreich war
■ restliche 96 *µ*l mit PCR-Aufreinigungs-Kit (von der Firma Qiagen) reinigen und letztlich mit 50 *µ*l destilliertem Wasser eluieren
■ zu den 50 *µ*l 5,7 *µ*l NEB 4 - Puffer und 1 *µ*l DpnI-Restriktionsendonukleas (20U) (von der Firma New England Biolabs) geben und 1 h verdauen
■ dann 6 *µ*l 3M Natrium-Acetat und 180 *µ*l (3 Vol.) 100% EtOH zugeben und 10 min auf -80°C stellen
■ 15 min bei 14000 rpm abzentrifugieren und mit 70% EtOH waschen
■ Pellet in Speed-Vac trocknen und in 10 *µ*l Wasser lösen

### DH10B mit BAC-Plasmid und pBAD-Plasmid elektrokompetent kompetent machen:

### Bakterien hochziehen und Arabinose-Induktion:

■ einzelne Bakterien-Kolonie (DH10B - mit MCMV-BAC Plasmid pSM3fr) picken und in 5 ml über Nacht bei 37°C (mit 25 *µ*g/ml Chloramphenicol (CAM) und 100 *µ*g/ml Ampicillin (Amp)) inkubieren
■ 250 ml LB-Medium (auch mit CAM und Amp) im Verhältnis 100:1 mit Bakterien-Kultur, die über Nacht inkubiert wurde (also 2,5 ml), animpfen
■ ca. 1 - 1,5 h bei 37°C wachsen lassen (bis OD₆₀₀ = 0,1-0,15)
■ dann frisch hergestellte 10% (w/v) Arabinose (von der Firma Sigma, Bestellnr. A3256) in LB-Medium zu Endkonzentration von 0,1 % zugeben
■ dann 40 min bis 1,5 h Bakterien weiter wachsen lassen bis OD₆₀₀ = 0,25 - 0,3

### Kompetent-Machen: (alles auf Eis und bei maximal 0 °C!)

■ pro 250 ml Bakterien-Kultur braucht man 750 ml 10% Glycerin-Lösung; diese muss sehr kalt sein; mindestens 3 h vorher in Eismaschine stellen
■ 250 ml Bakterien 15 min auf Eis
■ 10 min bei 7000 rpm abzentrifugieren
■ Pellet in 10 ml 10% Glycerin lösen und dann den ganzen 250 ml-Becher mit 10% Glycerin auffüllen
■ 10 min bei 7000 rpm abzentrifugieren
■ Pellet wieder resuspendieren und nochmal 10 min bei 7000 rpm abzentrifugieren
■ wieder resuspendieren und bei 7000 rpm 10 min zentrifugieren (insgesamt also 3 mal mit 10% Glycerin waschen)
■ dieses mal nach Abschütten des Überstands mit Kleenex-Tuch Becher auswischen
■ die paar 100 *µ* l an 10% Glycerin die noch im Becher vorhanden sind, reichen zum resuspendieren meist aus (allenfalls 100 *µ*l 10% Glycerin noch dazugeben)
■ 60 *µ* l Aliquots in Eppis und diese in füssigem. N₂ einfrieren

### Transformation mit PCR-Fragment:

■ 2 *µ*l PCR-Fragment in 60 *µ*l komp. Bakterien geben und dann sofort elektroporieren
■ Bedingungen: 2,5 kV, 200 Ohm, 25 *µ*FAD
■ dann 1ml LB-Medium dazugeben und 1,5 bis 2 h bei 37°C schütteln
■ kurz abzentrifugieren und in ca. 150 *µ*l LB resuspendieren und Ausstreichen auf LB-Platten mit 25 *µ*g/ml Kanamycin und 25 *µ*g/ml Chloramphenicol
■ Über Nacht bei 37°C wachsen lassen

### Charakterisierung des rekombinanten ie2-LCMV-GP-MCMV-BAC

Nach bekannten Standardprotokollen wurde DNA-Minipräparationen von 10 der erzielten ie2-LCMV-GP-BAC-Kolonien präpariert. Von 4 Klonen wurde eine DNA-Maxipräparation durchgeführt und die DNA mit Restriktionsendonuklease-Verdau analysiert. Die Analyse eines Klones ist exemplarisch in Abb. 15 gezeigt. Erfolgreiche Insertion des Epitops wird durch das Verschwinden der 18 kbp-Bande und des zusätzlichen Erscheinens einer 13 kbp- und einer 6 kbp-Bande (erstere ist nur als Doppelbande zu sehen) angezeigt.

Die korrekte Insertion des Epitops im richtigen Leseraster zum ie2-Gen des MCMV-Genoms wurde zusätzlich durch Sequenzierung bestätigt.
Für die Sequenzierung wurde folgender Primer verwendet, der in der neben dem Epitop bei der Mutagenese zusätzlich inserierten Kanamycin-Kassette bindet: 5`-GCAGACAGTTTTATTGTTCATGATG Die bei der Sequenzierung erhaltene Sequenz bestätigte die korrekte Insertion.

### Annex zu den Anmeldungsunterlagen - nachgereichtes Sequenzprotokoll

## Patentansprüche

1. Vektorsystem zum Einbringen von DNA in eukaryonte Zellen, umfassend Bakterien, die von eukaryonten Zellen aufgenommen werden oder aktiv in eukaryonte Zellen eindringen ("invasive Bakterien"), wobei die Bakterien ein eine heterologe DNA enthaltendes bakterielles künstliches Chromosom ("BAC") umfassen.

2. Vektorsystem nach Anspruch 1, **dadurch gekennzeichnet, daß** die Bakterien ausgewählt sind aus Stämmen der Gattungen Yersinia, Shigella, Salmonella und Escherichia.

3. Vektorsystem nach Anspruch 1, **dadurch gekennzeichnet, daß** die Bakterien ein oder mehrere Fremdgen(e) enthalten, das dazu führt/die dazu führen, daß ein natürlicherweise nicht invasives Bakterium zu einem invasiven Bakterium wird.

4. Vektorsystem nach Anspruch 3, **dadurch gekennzeichnet, daß** das Fremdgen das Invasin-Gen von Yersinia pseudotuberculosis oder Yersinia enterocolitica ("inv"-Gen) und gegebenenfalls das Listeriolysin-O-Gen von Listeria monocytogenes ("hly"-Gen) ist.

5. Vektorsystem nach Anspruch 4, **dadurch gekennzeichnet, daß** das inv-Gen und gegebenenfalls das hly-Gen im Bakterium auf einem Plasmid enthalten sind.

6. Vektorsystem nach einem der Ansprüche 1 bis 5, **dadurch gekennzeichnet, daß** die eukaryonten Zellen tierische Zellen oder menschliche Zellen in Zellkultur oder einem Gesamtorganismus sind.

7. Vektorsystem nach einem der Ansprüche 1 bis 6, **dadurch gekennzeichnet, daß** die heterologe DNA Selektions- und/oder Markergene und/oder Gene für katalytische Zwecke enthält.

8. Vektorsystem nach einem der Ansprüche 1 bis 7, **dadurch gekennzeichnet, daß** die in dem BAC enthaltene heterologe DNA virale DNA ist.

9. Vektorsystem nach Anspruch 8, **dadurch gekennzeichnet, daß** die virale DNA die DNA eines DNA-Virus ist oder eine DNA-Kopie der RNA eines RNA-Virus ist.

10. Vektorsystem nach Anspruch 9, **dadurch gekennzeichnet, daß** die virale DNA die DNA eines Herpes-Virus ist.

11. Vektorsystem nach Anspruch 10, **dadurch gekennzeichnet, daß** das Herpes-Virus das Cytomegalovirus (CMV-Virus) ist.

12. Vektorsystem nach einem der Ansprüche 8 bis 11, **dadurch gekennzeichnet, daß** die virale DNA infektiös oder replikationskompetent ist.

13. Vektorsystem nach einem der Ansprüche 8 bis 11, **dadurch gekennzeichnet, daß** die virale DNA nicht infektiös oder replikationskompetent ist.

14. Vektorsystem nach einem der Ansprüche 8 bis 13, **dadurch gekennzeichnet, daß** die virale DNA von identischen Sequenzabschnitten flankiert ist.

15. Vektorsystem nach einem der Ansprüche 8 bis 14, **dadurch gekennzeichnet, daß** die virale DNA zusätzlich weitere DNA-Abschnitte enthält.

16. Vektorsystem nach Anspruch 15, **dadurch gekennzeichnet, daß** dieser weitere DNA-Abschnitt für ein oder mehrere zusätzliche Gen-Produkte codiert.

17. Vektorsystem nach Anspruch 16, **dadurch gekennzeichnet, daß** das oder die zusätzliche(n) Gen-Produkt(e) ausgewählt ist/sind aus Wachstumsfaktoren, Hormonen, Enzymen, Impfantigenen, Cytotoxinen, immunmodulatorischen Proteinen oder Derivaten davon, Antisense-RNA-Molekülen und Ribozymen.

18. Vektorsystem nach Anspruch 15, **dadurch gekennzeichnet, daß** der zusätzliche DNA-Abschnitt im gleichen Leseraster in ein virales Gen integriert ist oder in Fusion mit einem viralen Gen vorliegt und für ein immunogenes Epitop codiert.

19. Verfahren zur Herstellung eines Vektorsystems nach einem der Ansprüche 1 bis 18, **dadurch gekennzeichnet, daß** man ein wie in einem der Ansprüche 1 bis 5 definiertes Bakterium mit einem BAC transformiert.

20. Zusammensetzung insbesondere in fester oder flüssiger Form, enthaltend ein Vektorsystem nach einem der Ansprüche 1 bis 19, gegebenenfalls zusammen mit üblichen pharmazeutischen Hilfsstoffen.

21. Vektorsystem nach einem der Ansprüche 1 bis 18 oder Zusammensetzung nach Anspruch 20 zur Verwendung als Impfstoff oder Arzneimittel.

22. Verwendung eines Vektorsystems nach einem der Ansprüche 1 bis 18 oder einer Zusammensetzung nach Anspruch 20 als Impfstoff oder Arzneimittel.

23. Eukaryonte Zelle, enthaltend ein Vektorsystem nach einem der Ansprüche 1 bis 19.

24. Verfahren zum Einbringen von DNA in eukaryonte Zellen, **dadurch gekennzeichnet, daß** eukaryonte Zellen mit einem Vektorsystem nach einem der Ansprüche 1 bis 18 oder einer Zusammensetzung nach Anspruch 20 in Kontakt gebracht werden.

25. Verfahren zur Charakterisierung von Virusmutanten, umfassend die folgenden Stufen:
(i) Einbringen eines oder mehrerer Gene(s), das/die dazu führt/führen, daß ein natürlicherwiese nicht-invasives Bakterium zu einem invasiven Bakterium wird, in Bakterien, die eine Bank randomisiert mutagenisierter viraler Genome in einem bakteriellen künstlichen Chromosom (BAC) enthalten;
(ii) Vermehrung der in Stufe (i) erhaltenen Bakterien;
(iii) Selektion auf Bakterien, die sowohl das inv-Gen und gegebenenfalls das hly-Gen als auch randomisiert mutagenisierte virale Genome in einem BAC enthalten;
(iv) Zugabe der in Stufe (iii) erhaltenen einzelnen Bakterienclone zu eukaryonten Zellen;
(v) Selektion lebensfähiger Viren;
(vi) Analyse der so erhaltenen Viren in einem geeigneten Testsystem.

26. Verfahren nach Anspruch 25, **dadurch gekennzeichnet, daß** anstelle der Stufe (i) Bakterien mit einem ein virales Genom enthaltenden BAC transformiert werden, wobei die Bakterien invasive Bakterien sind, und dann die das virale Genom enthaltenden BACs nach der Transformation randomisiert mutagenisiert werden.

27. Verfahren zum ortsspezifischen Einfügen eines heterologen DNA-Abschnittes in Virus-DNA enhaltende BACs, umfassend die folgenden Stufen:
(i) Durchführen einer PCR unter Verwendung eines Senseprimers A und eines Antisenseprimers B, wobei die 5'-Enden der Primer A und B homolog zu der Zielregion der Virus-DNA im BAC sind, in die der heterologe DNA-Abschnitt eingeführt werden soll, und die 3'-Enden der Primer A und B homolog zu den Enden des einzuführenden DNA-Abschnittes sind, wobei als Matrize für die PCR eine DNA verwendet wird, die den einzuführenden DNA-Abschnitt enthält;
(ii) Einbringen des in (i) enthaltenen PCR-Produktes und Virus enthaltender BACs in beliebiger Reihenfolge in Bakterien;
(iii) Selektion auf Rekombinanten zwischen PCR-Fragment und Virus-DNA enhaltenen BACs.

28. Verfahren nach Anspruch 27, **dadurch gekennzeichnet, daß** der eingeführte DNA-Abschnitt ein Gen, das für einen Selektionsmarker codiert, und gegebenenfalls ein weiteres DNA-Fragment, enthält.

29. Verfahren nach Anspruch 28, **dadurch gekennzeichnet, daß** das Gen, das für einen Selektionsmarker codiert, von einer loxP-Sequenz oder FRT-Sequenz flankiert ist.

30. Verfahren nach einem der Ansprüche 28 bis 29, **dadurch gekennzeichnet, daß** das weitere DNA-Fragment ein Gen ist, das für ein therapeutisches oder immunogenes Genprodukt codiert.

31. Verfahren nach einem der Ansprüche 27 bis 30, **dadurch gekennzeichnet, daß** einer der Primer A oder B zwischen dem Bereich, der homolog zur Zielregion der Virus-DNA ist, und dem Bereich, der homolog zur einzuführenden DNA ist, eine zusätzliche Fremd-DNA-Sequenz enthält.

32. Verfahren nach Anspruch 31, **dadurch gekennzeichnet, daß** die Fremd-DNA-Sequenz für ein immunogenes Epitop oder kurzes Peptid codiert.

33. Verfahren nach Anspruch 32, **dadurch gekennzeichnet, daß** die Primersequenzen so gewählt sind, daß im rekombinanten BAC die DNA-Sequenz für das immunogene Epitop oder das kurze Peptid im gleichen Leseraster liegt wie ein virales Gen.

34. Verfahren nach einem der Ansprüche 27 bis 33, **dadurch gekennzeichnet, daß** es sich bei der Virus-DNA um eine Herpes-Virus-DNA handelt.

35. Verfahren nach einem der Ansprüche 31 bis 34, **dadurch gekennzeichnet, daß** es sich bei den Bakterien um E. coli handelt, insbesondere um E. coli das die Gene redα, redβ und redγ exprimiert und gegebenenfalls recA⁻ ist.

36. Verfahren nach einem der Ansprüche 27 bis 35, **dadurch gekennzeichnet, daß** die Primer A und B jeweils in einem Bereich von mindestens 25 bp zu der Zielsequenz in der viralen DNA homolog sind.


SEQUENZPROTOKOLL

<110> Prof. Dr. Koszinowski, Ulrich

<120> Vektorsystem zum Einbringen von DNA in eukaryonte
      Zellen

<130> 11581

<140> EP 01 109 460.4
<141> 2001-04-24

<150> DE 100 20 500.3
<151> 2000-04-26

<160> 6

<170> PatentIn Ver. 2.1

<210> 1
<211> 55
<212> DNA
<213> genomisch

<400> 1
gactcgacgg cgtcgtcaaa caaggcgtcg cgccgctgtc gcgccccgcg cggaa      55

<210> 2
<211> 58
<212> DNA
<213> Künstliche Sequenz

<220>
<223> Beschreibung der künstlichen Sequenz: Primer

<400> 2
gaaccacggg ttctttctct tgaccagaga cctggtgacc gtcaggaaga agattcag   58

<210> 3
<211> 22
<212> DNA
<213> Künstliche Sequenz

<220>
<223> Beschreibung der künstlichen Sequenz: Primer

<400> 3
cgatttattc aacaaagcca cg                                                22

<210> 4
<211> 50
<212> DNA
<213> Künstliche Sequenz

<220>
<223> Beschreibung der künstlichen Sequenz: Primer

<400> 4
ctgtccgatg aataaaacct ctttatttat tgattaaaaa ccatgacata                  50

<210> 5
<211> 35
<212> DNA
<213> Künstliche Sequenz

<220>
<223> Beschreibung der künstlichen Sequenz: Primer

<400> 5
cctcgtgtcc tcgccagtgt tacaaccaat taacc                                  35

<210> 6
<211> 25
<212> DNA
<213> Künstliche Sequenz

<220>
<223> Beschreibung der künstlichen Sequenz: Primer

<400> 6
gcagacagtt ttattgttca tgatg                                             25